# EUROPEAN PATENT SPECIFICATION

(11) **EP 4 136 659 B1**
(45) Date of publication and mention of the grant of the patent: **04.12.2024**
(21) Application number: 21788257.0
(22) Date of filing: 16.04.2021
(51) Int. Cl.: A61B 5/11, A61B 5/00, A61B 5/053, A61B 5/313, A61B 5/389, G16H 20/30, G16H 50/20, G16H 50/30

(54) **METHOD OF GENERATING AN INDICATION OF MUSCLE FATIGUE**
VERFAHREN ZUR ERZEUGUNG EINER ANZEIGE VON MUSKELERMÜDUNG
PROCÉDÉ DE GÉNÉRATION D'UNE INDICATION DE FATIGUE MUSCULAIRE

(30) Priority: 16.04.2020 US 202063010868 P
(43) Date of publication of application: 22.02.2023
(73) Proprietor: Université Laval, Québec City, Québec G1V 0A6 (CA)
(72) Inventor: MESSADDEQ, Younès, Québec, Québec G2K 2C2 (CA); GOSSELIN, Benoit, Québec G3B 0E8 (CA); ROUDJANE, Mourad, Québec, Québec G1V 2T1 (CA); BOUYER, Laurent, Québec, Québec G1Y 3S5 (CA); GAUTHIER, Nicolas, Lévis, Québec G6X 2Z7 (CA); BIELMANN, Mathieu, Québec, Québec G1M 2S8 (CA); GAGNON-TURCOTTE, Gabriel, Québec, Québec G1H 1E7 (CA)
(74) Representative: Plasseraud IP
(86) International application number: PCT/CA2021/050519
(87) International publication number: WO 2021/207852

(56) References cited:
- WO-A1-2012/071551
- WO-A1-2019/134552
- CN-A- 110 338 808
- CN-A- 110 338 808
- US-A1- 2001 048 482
- US-A1- 2001 049 482
- US-A1- 2016 051 183
- US-A1- 2020 029 882
- US-A1- 2020 029 882
- WONG AZMAN AMELIA ET AL: "ANALYSIS ON THE EFFECT OF STIMULATOR PARAMETERS IN ELECTRICAL STIMULATION PROCEDURE ON THE HUMAN BICEP MUSCLE", JURNAL TEKNOLOGI, vol. 78, no. 7-5, 28 March 2016 (2016-03-28), Malaysia, XP093096443, ISSN: 0127-9696, DOI: 10.11113/jt.v78.9446
- AL-MULLA MOHAMED R., SEPULVEDA FRANCISCO: "Novel Feature Modelling the Prediction and Detection of sEMG Muscle Fatigue towards an Automated Wearable System", SENSORS, vol. 10, no. 5, 1 January 2010 (2010-01-01), pages 4838 - 4854, XP055864496, DOI: 10.3390/s100504838

## Description

### TECHNICAL FIELD

This application relates to wearable technology, and more specifically to the acquisition of signals indicative of movement and/or muscular activity from the body of a mammal, and in particular from humans.

### BACKGROUND

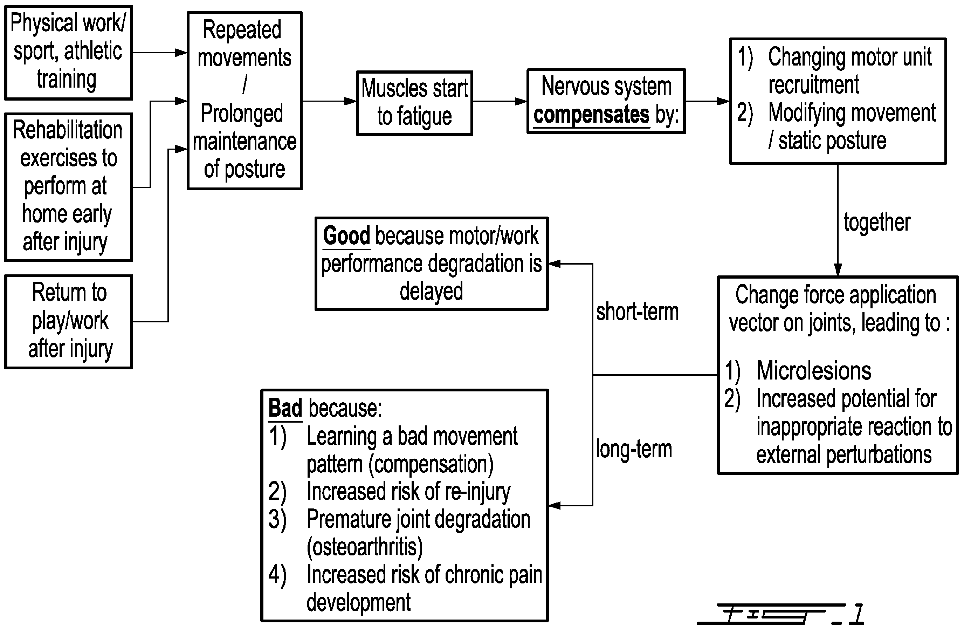

Fig. 1 schematically represents scenarios associated to muscle fatigue. There are many situations where muscle fatigue can occur, and where obtaining an objective indication of muscle fatigue could be useful to help avoid one or more of learning a bad movement, increased risk of injury or re-injury, premature joint degradation (osteoarthritis) and/or increased risk of chronic pain development. Such situations can include physical work, sport or athletic training, rehabilitation exercises to perform after injury, or returning to work or play after an injury, for instance. Monitoring an objective indication of muscle fatigue during such situations can also be useful in understanding the cause of a symptom such as one or more of recurring occurrence of a bad movement, injury or re-injury, premature joint degradation (osteoarthritis) and/or chronic pain development.

Indeed, such situations can involve repeated movements or prolonged maintenance of posture which can lead to muscle fatigue. As muscle fatigue begins occurring, the nervous system typically compensates by changing motor unit recruitment and modifying the movement or static posture, which can cause a change of force application vectors on joints, leading to micro lesions and increased potential for inappropriate reaction to external perturbations. In the short-term, the reaction can be good because it can delay motor/work performance degradation. However, in the long-term, the reaction can lead to undesired symptoms such as those indicated above.

For instance, work, sports, and field operation related musculoskeletal injuries (W/S/FO-MSKI) represent a major source of injury and a principal economic burden in modern societies and in military operations. While the etiology of these disorders is multifactorial, the gradual, unaware development of muscle fatigue is likely a major contributor. High intensity and/or repetitive movements may lead to a gradual fatigue of small muscle compartments ('motor units'). Muscle fatigue is normally experienced as a reduction in muscle force and/or the appearance of movement clumsiness. While the neural compensation is initially effective, it leads to a change in the way muscles pull on bones, thereby producing non-optimal force vectors around joints. With repetitive use, this may lead to early joint wear (osteoarthritis) or non-optimal response to unexpected perturbations, thereby increasing the risk of developing a musculoskeletal injury.

Similarly, upon return to work/play/field operations after injury, or during rehabilitation exercises performed early after injury, deconditioned muscles and pain can also lead to abnormal motor unit recruitment and put the person in a similar increased risk situation. Following of sport injury or work-related disease, patients often require a rehabilitation intervention. However, in real life conditions, it is challenging for health care professionals to acquire objective data and to monitor the performance of patients during the physical assessment. At the same time, laboratory assessment does not provide data accurately reflecting the reality of the patient's performance in real-life environments. Document US 2020/029882 A1 discloses a processor-implemented method for body analysis comprising a step of g obtaining data from a linear displacement sensor wherein the linear displacement sensor comprises a strip of stretchable electroactive polymer. Document CN 110 338 808 A discloses a sensor patch for sport motion detection wherein the patch includes both a tensile strain sensor layer and surface EMG detection electrodes. Document WO 2012/071551 A1 discloses a computer-implemented method for estimating with a processor a muscle fatigue value by comparing an obtained form profile against a first profile template for an athletic movement.

There remained room for improvement in terms of methods and systems for providing efficient, objective indications of muscle fatigue, in practical ways, and at an accessible cost.

### SUMMARY

The invention is defined by the appended claims. The following lists aspects of the disclosure. In accordance with one aspect, there is provided a computer-implemented method of generating a composite indication of muscle fatigue of a mammal, the method comprising : acquiring measured sEMG median frequency values of successive portions of a sEMG signal within corresponding successive time windows, the sEMG signal being of muscular activity of the mammal, the muscular activity entraining a movement relative to a joint of the mammal; generating a first indication of muscle fatigue based on a comparison between at least one of the measured sEMG median frequency value and a reference median frequency value;acquiring a movement signal indicative of the movement; generating a second indication of muscle fatigue based on said movement signal; and generating a composite indication of muscle fatigue based on both the first indication of muscle fatigue and the second indication of muscle fatigue.

In accordance with the claimed invention, there is provided a method of generating an indication of muscle fatigue of a mammal wearing a fiber of an electrically conductive polymer material over a muscle having muscle fibers, the fiber extending along a fiber path having a length and a width, the length of the fiber path extending along a length of the fibers of the muscle, the method comprising:
the muscle deforming the fiber path including activating a joint of the mammal; circulating electricity along the fiber, along the length of the fiber path; generating a movement signal including monitoring a change of impedance of the fiber stemming from the deformation of the muscle path during said deformation of the fiber path; determining a coefficient of correlation value of successive portions of the movement signal associated to corresponding, successive, time windows, including ascertaining a degree of similitude between the corresponding portion and a corresponding movement template; generating an indication of muscle fatigue based on said coefficient of correlation values.

In accordance with another aspect, there is provided a computer-implemented method of generating an indication of muscle fatigue of a mammal, the method comprising : acquiring a movement signal stemming from a fiber deforming upon movement of the mammal; measuring coefficient of correlation values of successive portions of the movement signal within corresponding time windows, the measured coefficient of correlation values representative of a degree of similarity between the successive portions of the movement signal and a corresponding movement template, generating an indication of muscle fatigue based on said measured coefficient of correlation values.

Said generating an indication of muscle fatigue can be based on a comparison between at least one of the measured coefficient of correlation values and a reference coefficient of correlation value.

The reference coefficient of correlation value can be a threshold coefficient of correlation value, wherein said generating an indication of muscle fatigue can be contingent upon on said measured coefficient of correlation value exceeding the threshold coefficient of correlation value.

The method can further comprise measuring a variability of said coefficient of correlation values over the successive portions of the movement signal, said generating an indication of muscle fatigue can be based on a comparison between at least one of said measured variability value and a reference variability value.

The reference variability value can be a threshold variability value, said generating a second indication of muscle fatigue being contingent upon said at least one measured variability value exceeding the threshold variability value.

The movement can be a repeated movement sequence, the method can further comprise recognizing individual ones of the movements in the sequence, and defining the time windows in a manner to match a corresponding time window to each one of the movements.

The method can further comprise defining the movement template based on one or more of said repeated movements.

The time windows can have a same, predetermined period of time and be immediately one after the other.

The method can further comprise defining the reference coefficient of correlation value based on one or more of said measured coefficient of correlation values.

The method can further comprise defining the reference variability value based on one or more measured variability values.

In accordance with another aspect, a computer program product stored in a non-transitory computer readable memory and operative to, when operated upon by a processor, perform the method.

In accordance with another aspect, there is provided a sensor for monitoring muscle activity of a mammal, the sensor comprising:
an apparel wearable by a mammal in a manner to cover skin of the mammal, the covered skin extending over muscle of the mammal, the muscle having muscle fibers; a fiber of an electrically conductive polymer material, the fiber having a portion of its length forming a first electrode, the fiber secured to the apparel along a fiber path in a manner that the fiber path is deformable by deformation of the apparel, the fiber path having a length and a width defined relative the apparel in a manner that when the garment is worn by the mammal, i) the length of the fiber path extends along a length of the fibers of the muscle, the fiber path deforming when the muscle activates a joint of the mammal, ii) the portion forming the first electrode extends transversally relative to an orientation of muscle fibers of the muscle and is maintained into electrical contact with the skin by the apparel, and a second electrode secured to the apparel, spaced apart from the first electrode along the orientation of muscle fibers; and a first electrical connection to the portion of the fiber forming the first electrode, a second electrical connection to a point of the fiber spaced apart from the portion forming the first electrode along the length of the fiber path, and a third electrical connection to the second electrode.

The sensor can further comprise a third electrode secured to the apparel, and a third electrical lead connecting the third electrode.

The second electrode can be provided in the form of an other portion of the length of the fiber.

The fiber can be a first fiber, the sensor can further comprise a second fiber also having a movement detection portion, the other one of the electrodes being provided in the form of an electrode portion of the length of the second fiber.

The first fiber and the second fiber can be connected in a half full Wheatstone bridge configuration, the other one of the electrodes can be provided in the form of an electrode portion of the length of the second fiber.

The movement detection portions of the first and second fibers can have a same resistance and be secured to the apparel in a manner to deforming equally upon the movement.

A length of the fiber extending between the first electrode and the point connected by the second electrical lead can have a sinuous shape including a plurality of alternatingly oriented, transversally oriented curves.

The apparel can have an adhesive face covered by a removable layer and can be configured to be worn by the mammal by adhering its adhesive face to the skin of the mammal following removal of the removable layer.

The apparel can have an elastic band, and can be configured to be worn by the mammal by stretching the elastic band and releasing the elastic band in a manner to compressively trap a portion of mammal having the muscle therein.

The fiber can have at least 15 cm in length, preferably between 20 and 40 cm in length.

The sensor can further have a DC canceler secured to the apparel and forming part of the electrical connections.

The sensor can further have an interrogation system having an AC electrical drive configured to circulate electricity along an AC electrical drive circuit including the movement detection portion, a movement acquirer including an impedance meter associated to the AC electrical drive circuit and configured to measure a reaction of the circuit to the change of resistance of the fiber and generate a movement signal based on the measurement, a sEMG acquirer connected to the electrode portion and to another one of the electrodes to acquire a sEMG signal therebetween.

The AC electrical drive can have a frequency outside a sEMG frequency bandwidth of at least 15-250Hz, at least 10-350Hz, or at least 0-600Hz.

The sensor can further comprise a DC canceler in the AC electrical drive circuit.

The sensor can further comprise a filter shielding the sEMG acquirer from a frequency of the AC electrical drive.

The sensor can further comprise a filter shielding the movement acquirer from a sEMG bandwidth.

The sEMG acquirer can include reference voltage setpoint connectable to yet another one of the electrodes.

The sensor can further comprise at least one analog to digital converter operatively connected to convert the sEMG signal and the movement signal from analog to digital.

The sensor can further comprise a transmitter configured to emit a signal based on the sEMG signal and on the movement signal.

The sEMG acquirer and/or the movement acquirer can include an amplifier.

The movement acquirer and the sEMG acquirer can share a connection to the electrode portion and to the second electrode, the sensor can further comprise a filter to direct a movement frequency bandwidth to the movement meter, and to direct a sEMG bandwidth excluding the movement frequency bandwidth to a sEMG differential voltage meter.

The fiber can be a first fiber, the sensor can further comprise a second fiber also having a movement detection portion, the other one of the electrodes can be provided in the form of an electrode portion of the length of the second fiber, and the AC electrical drive circuit can be connectable to include the movement detection portions of both the first fiber and the second fiber and to circulate electricity equally along both movement detection portions, the movement detection portions having a same resistance and deforming equally by the target movement.

The electrode portion can be a first electrode portion, the second can be in the form of a second electrode portion of the length of the fiber, the AC electrical drive can have an oscillator having a resonance frequency modulated by the change of resistance, and the impedance meter can be configured to measure the resonance frequency.

The sensor can further comprise a coefficient generator configured to measure coefficient of correlation values of successive portions of the movement signal within corresponding time windows, the measured coefficient of correlation values representative of a degree of similarity between the successive portions of the movement signal and a corresponding movement template.

The sensor can further comprise a coefficient indication generator configured to generate a coefficient indication of muscle fatigue can be based on said measured coefficient of correlation values.

Generating the coefficient indication of muscle fatigue can be based on a comparison between at least one of the measured coefficient of correlation values and a reference coefficient of correlation value.

The reference coefficient of correlation value can be a threshold coefficient of correlation value, the generating a coefficient indication of muscle fatigue can be contingent upon on said measured coefficient of correlation value exceeding the threshold coefficient of correlation value.

The sensor can further comprise a variability determinator configured to measure a variability of said coefficient of correlation values over the successive portions of the movement signal, and a variability indication generator configured to generate an variability indication of muscle fatigue based on a comparison between at least one of said measured variability value and a reference variability value.

The variability value can be a threshold variability value, said generating a variability indication of muscle fatigue being contingent upon said at least one measured variability value exceeding the threshold variability value.

The sensor can further comprise a median frequency calculator configured to measure sEMG median frequency values of successive portions of a sEMG signal within corresponding successive time windows.

The sensor can have a sEMG indication generator configured to generate a sEMG indication of muscle fatigue based on a comparison between at least one of the measured sEMG median frequency value and a reference median frequency value.

The reference median frequency value can be a threshold reference median frequency value, the generating the sEMG indication of muscle fatigue of muscle fatigue can be contingent upon said sEMG median frequency value exceeding the threshold reference median frequency value.

The sensor can further comprise a composite indication generator configured to trigger an alarm from a user interface contingent upon the sEMG indication of muscle fatigue and further based on the measured coefficient of correlation values.

The targeted movement can be a repeated movement sequence, wherein the interrogation system further comprise a window determinator configured for recognizing individual ones of the movements in the sequence, and defining the time windows in a manner to match a corresponding time window to each one of the movements.

The movement template can be constructed based on one or more of said repeated movements.

The time windows can have a same, predetermined period of time and be immediately one after the other.

The sEMG indication generator can be further configured to define the reference median frequency value based on one or more of said measured sEMG median frequency values.

The correlation indication generator can be further configured to define the reference coefficient of correlation value based on one or more of said measured coefficient of correlation values.

The variability indication generator can be further configured to define the reference variability value based on one or more of the measured variability values.

In accordance with another aspect, there is provided a system for interrogating a sensor having a fiber of an electrically conductive material wearable in a manner for a movement detection portion of the length of the fiber to be deformed by a target movement, the resistance of the fiber deforming upon said deformation, and a plurality of electrodes, at least one of the electrodes being provided in the form of an electrode portion of the length of the fiber, the system comprising : an AC electrical drive configured to circulate electricity along an AC electrical drive circuit connectable to include the movement detection portion, a movement acquirer including an impedance meter associated to the AC electrical drive circuit and configured to measure a reaction of the circuit to the change of resistance of the fiber and generate a movement signal based on the measurement, and a sEMG acquirer connectable to the electrode portion and to another one of the electrodes to acquire a sEMG signal therebetween.

The AC electrical drive can have a frequency outside a sEMG frequency bandwidth of at least 15-250Hz, at least 10-350Hz, or of at least 0-600Hz.

The system can further comprise a DC canceler in the AC electrical drive circuit.

The system can further comprise a filter shielding the sEMG acquirer from a frequency of the AC electrical drive.

The system can further comprise a filter shielding the movement acquirer from a sEMG bandwidth.

The sEMG acquirer can include a reference voltage setpoint connectable to yet another one of the electrodes.

The system can further comprise at least one analog to digital converter operatively connected to convert the sEMG signal and the movement signal from analog to digital.

The system can further comprise a transmitter configured to emit a signal based on the sEMG signal and on the movement signal.

The sEMG acquirer and/or the movement acquire can include an amplifier.

The movement acquirer and the sEMG acquirer can share a connection to the electrode portion and to the other electrode, the system can further comprise a filter to direct a movement frequency bandwidth to the movement meter, and to direct a sEMG bandwidth excluding the movement frequency bandwidth to a sEMG differential voltage meter.

The system can further comprise the sensor, wherein the fiber is a first fiber, the sensor can further comprise a second fiber also having a movement detection portion, the first fiber and the second fiber being in a half full Wheatstone bridge configuration, the other one of the electrodes being provided in the form of an electrode portion of the length of the second fiber.

The fiber can be a first fiber, the sensor can further comprise a second fiber also having a movement detection portion, the other one of the electrodes being provided in the form of an electrode portion of the length of the second fiber.

The AC electrical drive circuit can be connectable to include the movement detection portions of both the first fiber and the second fiber and to circulate electricity equally along both movement detection portions, the movement detection portions having a same resistance and deforming equally by the target movement.

The electrode portion can be a first electrode portion, the other electrode being provided in the form of a second electrode portion of the length of the fiber, the AC electrical drive can have an oscillator having a resonance frequency modulated by the change of resistance, and the impedance meter can be configured to measure the resonance frequency.

The system can further comprise a coefficient generator configured to measure coefficient of correlation values of successive portions of the movement signal within corresponding time windows, the measured coefficient of correlation values representative of a degree of similarity between the successive portions of the movement signal and a corresponding movement template.

The system can further comprise a coefficient indication generator configured to generate a coefficient indication of muscle fatigue can be based on said measured coefficient of correlation values.

Generating the coefficient indication of muscle fatigue can be based on a comparison between at least one of the measured coefficient of correlation values and a reference coefficient of correlation value.

The reference coefficient of correlation value can be a threshold coefficient of correlation value, said generating a coefficient indication of muscle fatigue can be contingent upon on said measured coefficient of correlation value exceeding the threshold coefficient of correlation value.

The system can further comprise a variability determinator configured to measure a variability of said coefficient of correlation values over the successive portions of the movement signal, and a variability indication generator configured to generate an variability indication of muscle fatigue based on a comparison between at least one of said measured variability value and a reference variability value.

The reference variability value can be a threshold variability value, said generating a variability indication of muscle fatigue being contingent upon said at least one measured variability value exceeding the threshold variability value.

The system can further comprise a median frequency calculator configured to measure sEMG median frequency values of successive portions of a sEMG signal within corresponding successive time windows.

The system can further comprise a sEMG indication generator configured to generate a sEMG indication of muscle fatigue based on a comparison between at least one of the measured sEMG median frequency value and a reference median frequency value.

The reference median frequency value can be a threshold reference median frequency value, the generating the sEMG indication of muscle fatigue of muscle fatigue being contingent upon said sEMG median frequency value exceeding the threshold reference median frequency value.

The system can further comprise a composite indication generator configured to trigger an alarm from a user interface contingent upon the sEMG indication of muscle fatigue and further based on the measured coefficient of correlation values.

The targeted movement can be a repeated movement sequence, the system can further comprise a window determinator configured for recognizing individual ones of the movements in the sequence, and defining the time windows in a manner to match a corresponding time window to each one of the movements. The movement template can be defined based on one or more of said repeated movements. The time windows can have a same, predetermined period of time and are immediately one after the other.

The sEMG indication generator can be further configured to defining the reference median frequency value based on one or more of said measured sEMG median frequency values.

The correlation indication generator can be further configured to define the reference coefficient of correlation value based on one or more of said measured coefficient of correlation values.

The variability indication generator can be further configured to define the reference variability value based on one or more of the measured variability values.

In accordance with another aspect, there is provided a method of interrogating a sensor having a fiber of an electrically conductive material wearable in a manner for a movement detection portion of the length of the fiber to be deformed by a target movement, the resistance of the fiber deforming upon said deformation, and a plurality of electrodes, at least one of the electrodes being provided in the form of an electrode portion of the length of the fiber, the method comprising : circulating AC electricity along an AC electrical drive circuit including the movement detection portion, measuring a reaction of the circuit to the change of resistance of the fiber, thereby generating a movement signal, acquiring a sEMG signal from the electrode portion and another one of the electrodes.

The AC electricity can have a frequency outside a sEMG frequency bandwidth of at least 15-250Hz, at least 10-350Hz, or of at least 0-600Hz.

The method can further comprise cancelling any DC component in the AC electrical drive circuit.

The method can further comprise shielding the sEMG acquisition from a frequency of the AC electrical drive.

The method can further comprise shielding the movement signal from a sEMG bandwidth.

The method can further comprise imparting a reference voltage setpoint to yet another one of the electrodes.

The method can further comprise converting the sEMG signal and the movement signal from analog to digital.

The method can further comprise a emitting a signal based on both the sEMG signal and on the movement signal.

The method can further comprise amplifying the sEMG signal.

The method can further comprise amplifying the movement signal.

The method can further comprise splitting the AC frequency from the sEMG bandwidth.

The method can further comprise the target movement deforming the sensor, wherein the fiber can be a first fiber, the sensor can further comprise a second fiber also having a movement detection portion, the first fiber and the second fiber being in a half full Wheatstone bridge configuration, the other one of the electrodes being provided in the form of an electrode portion of the length of the second fiber, the method can further comprise acquiring a signal including the movement signal superposed to the sEMG signal from the half full Wheatstone bridge configuration.

The method can further comprise the target movement deforming the sensor wherein the fiber can be a first fiber, the sensor method can further comprise a second fiber also having a movement detection portion, the other one of the electrodes being provided in the form of an electrode portion of the length of the second fiber.

Circulating AC electricity can include circulating AC electricity equally along the movement detection portions of both the first fiber and the second fiber, the movement detection portions deforming and resisting to said electricity equally during the target movement.

The electrode portion can be a first electrode portion, the other electrode being provided in the form of a second electrode portion of the length of the fiber, wherein the AC electrical drive can have an oscillator having a resonance frequency modulated by the change of resistance, and measuring the resistance can include measuring the resonance frequency.

The method further comprises measuring coefficient of correlation values of successive portions of the movement signal within corresponding time windows, the measured coefficient of correlation values representative of a degree of similarity between the successive portions of the movement signal and a corresponding movement template.

The method further comprises generating a coefficient indication of muscle fatigue can be based on said measured coefficient of correlation values.

Generating the coefficient indication of muscle fatigue can include comparing at least one of the measured coefficient of correlation values to a reference coefficient of correlation value. The reference coefficient of correlation value can be a threshold coefficient of correlation value, wherein said generating a coefficient indication of muscle fatigue can be contingent upon on said measured coefficient of correlation value exceeding the threshold coefficient of correlation value.

The method can further comprise measuring a variability of said coefficient of correlation values over the successive portions of the movement signal, and generating an variability indication of muscle fatigue including comparing at least one of said measured variability value to a reference variability value. The reference variability value can be a threshold variability value, said generating a variability indication of muscle fatigue being contingent upon said at least one measured variability value exceeding the threshold variability value.

The method further comprises measuring sEMG median frequency values of successive portions of a sEMG signal within corresponding successive time windows. The method further comprises generating a sEMG indication of muscle fatigue based on a comparison between at least one of the measured sEMG median frequency value and a reference median frequency value. The reference median frequency value is a threshold reference median frequency value, the generating the sEMG indication of muscle fatigue of muscle fatigue being contingent upon said sEMG median frequency value falling below the threshold reference median frequency value.

The method can further comprise triggering an alarm from a user interface contingent upon the sEMG indication of muscle fatigue and further based on the measured coefficient of correlation values.

The targeted movement can be a repeated movement sequence, the method can further comprise recognizing individual ones of the movements in the sequence, and defining the time windows in a manner to match a corresponding time window to each one of the movements.

The method can further comprise defining the movement template based on one or more of said repeated movements.

The time windows can have a same, predetermined period of time and be immediately one after the other.

The method can further comprise defining the reference median frequency value based on one or more of said measured sEMG median frequency values.

The method can further comprise defining the reference coefficient of correlation value based on one or more of said measured coefficient of correlation values.

The method can further comprise defining the reference variability value based on one or more of the measured variability values.

In accordance with other aspects, there is provided a method, device and system to acquire a signal indicative of body movement compatible with the reality of wearable technology. There is also provided a method of obtaining an indication of fatigue and/or risk of injury on the basis of the signal indicative of body movement.

In accordance with other aspects, there is provided a method, device and system to acquire both a signal indicative of body movement and an EMG signal compatible with the reality of wearable technology. There is also provided a method of obtaining an indication of muscle fatigue and/or risk of injury on the basis of the combination of the signal indicative of body movement and the EMG signal. The latter method can be used as a basis of generating a signal warning a wearer of the device of muscle fatigue and/or a risk of injury, or to complete a report to later be communicated to an expert, for instance.

In accordance with one aspect, there is provided a system to acquire a signal indicative of movement of a mammal's body portion relative to an joint of the mammal, the system comprising a fiber having a resistance that varies upon deformation, the fiber being elongated, made of an electrically conductive material, and securable to the mammal's body portion in a manner to deform upon movement of the mammal's body portion, an electrical power source connected to circulate electricity along the length of the fiber, and a meter to measure a response signal of the fiber to the circulation of electricity.

It will be understood that the expression "computer" as used herein is not to be interpreted in a limiting manner. It is rather used in a broad sense to generally refer to the combination of some form of one or more processing units and some form of non-transitory memory system accessible by the processing unit(s). The use of the expression "computer" in its singular form as used herein includes within its scope the combination of a two or more computers working collaboratively to perform a given function. Moreover, the expression "computer" as used herein includes within its scope the use of partial capacities of a processing unit of an elaborate computing system also adapted to perform other functions. Similarly, the expression 'controller' as used herein is not to be interpreted in a limiting manner but rather in a general sense of a device, or of a system having more than one device, performing the function(s) of controlling one or more device such as an electronic device or an actuator for instance.

It will be understood that the various functions of a computer or of a controller can be performed by hardware or by a combination of both hardware and software. For example, hardware can include logic gates included as part of a silicon chip of the processor. Software can be in the form of data such as computer-readable instructions stored in the memory system. With respect to a computer or a controller, the expression "configured to" relates to the presence of hardware or a combination of hardware and software which is operable to perform the associated functions.

Many further features and combinations thereof concerning the present improvements will appear to those skilled in the art following a reading of the instant disclosure.

### DESCRIPTION OF THE FIGURES

In the figures,
Fig. 1 is a flow chart representing scenarios associated to muscle fatigue;
Fig. 2 is a flow chart representing the generation of a composite indication of muscle fatigue;
Fig. 3A is a block diagram of a sEMG acquirer;
Fig. 3B is a graph showing sEMG amplitude distribution over a frequency spectrum;
Fig. 4 includes graphs A and B representing an example sEMG signal in static and dynamic conditions, respectively and graphs C and D, representing a movement signal in static and dynamic conditions, respectively;

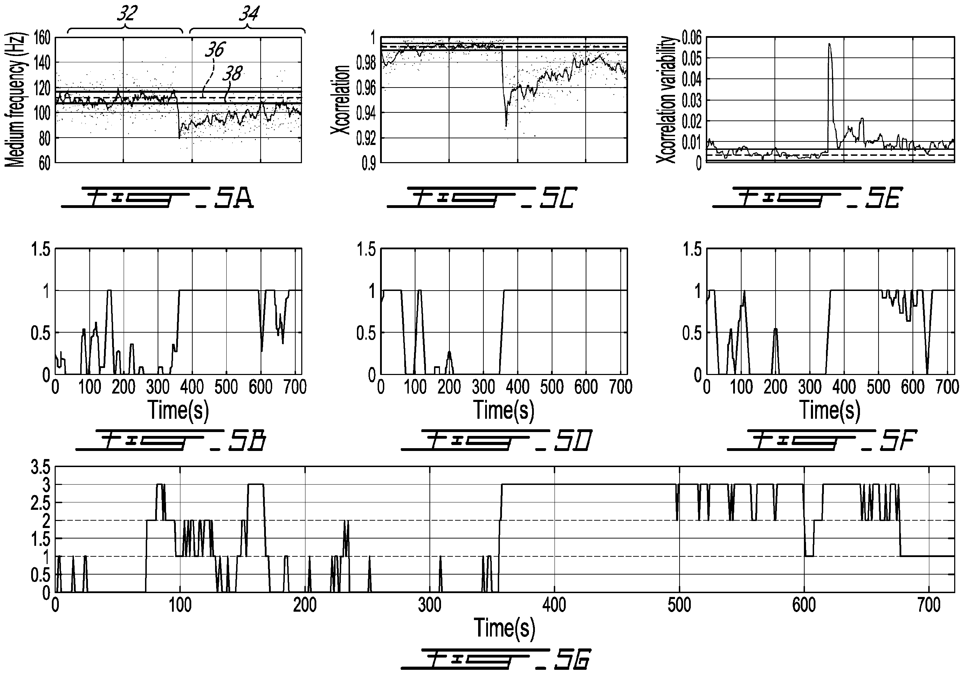

Fig. 5 includes graphs A and B representing the evolution of sEMG median frequency and the generation of a corresponding indication of muscle fatigue over time, respectively, graphs C and D representing the evolution of a coefficient of correlation of a movement signal and the generation of a corresponding indication of muscle fatigue over time, respectively, graphs E and F representing the evolution of a variability of the coefficient of correlation and the generation of a corresponding indication of muscle fatigue over time, respectively, and graph G represents the evolution of a composite indication of muscle fatigue over time, formed on the basis of the three indications of muscle fatigue;
Fig. 6A presents an example fiber worn on the shoulder of a user;
Fig. 6B is a block diagram of a movement acquirer;
Fig. 7 is a flow chart representing the generation of an indication of muscle fatigue based a movement signal;
Fig. 8A and 8B are graphs showing template matching and the evolution of a correlation coefficient over time, respectively, for three dynamic repeated movement scenarios including the hip (top), the knee (middle), and the ankle (bottom);
Fig. 9A, 9B and 9C are schematic diagrams showing example sensors;
Fig. 10 is a block diagram showing an example system for generating a composite indication of muscle fatigue;
Fig. 11A is a chart representing an sequence of steps for producing a fiber, whereas Fig. 11B are microscope images of an example fiber;
Figs 12A to 12D show various example sensor configurations each incorporating at least one fiber;
Fig. 13A and 13B are pictures showing two example of apparels serving as a support to the one or more fibers and electrodes for functional wearing by a user, with Fig. 13A representing an adhesive apparel and Fig. 13B representing a textile-based apparel;
Fig. 14 is an electronics schematic in accordance with one example embodiment;
Fig. 15A is an electronics schematic in accordance with another example embodiment;
Fig. 15B is an example circuit in accordance with the schematic of Fig. 15A; and
Fig. 16 is an electronics schematic in accordance with another example embodiment.

### DETAILED DESCRIPTION

As presented above, muscle fatigue can entrain variations in the muscle force or agility of muscle recruitment, as well as in movement such as by a noticeable change in movement. From a wearable sensor point of view, different signals can be used as a basis of forming an objective indication of muscle fatigue. Two of such signals will be discussed herein:
electromyography (EMG), and more specifically surface electromyography (sEMG), and movement monitoring. Indeed, muscle fatigue can entrain reduction of the median frequency of sEMG. Accordingly, monitoring the median frequency, and determining whether it has reduced in excess of a given threshold, can form the basis of one indicator of muscle fatigue. Comparably, muscle fatigue typically entrains an effect in the movement of the user. In a static posture, such as holding a heavy object with one hand and the arm oriented horizontally for instance, muscle fatigue typically entrains a weakening of the solicited muscle(s), and eventually causes lowering of the arm in the latter example. In a repeated dynamic movement, muscle fatigue can also frequently entrain some change in the movement over time, and measuring this variation, and determining if it has exceeded a given threshold, can form the basis of another indicator of muscle fatigue (and we will see that by monitoring movement, we can obtain more than one indicator of muscle fatigue, since a first indicator can be obtained by measuring a change in the movement, and a second indicator can be obtained by measuring a change in the variability of the movement).

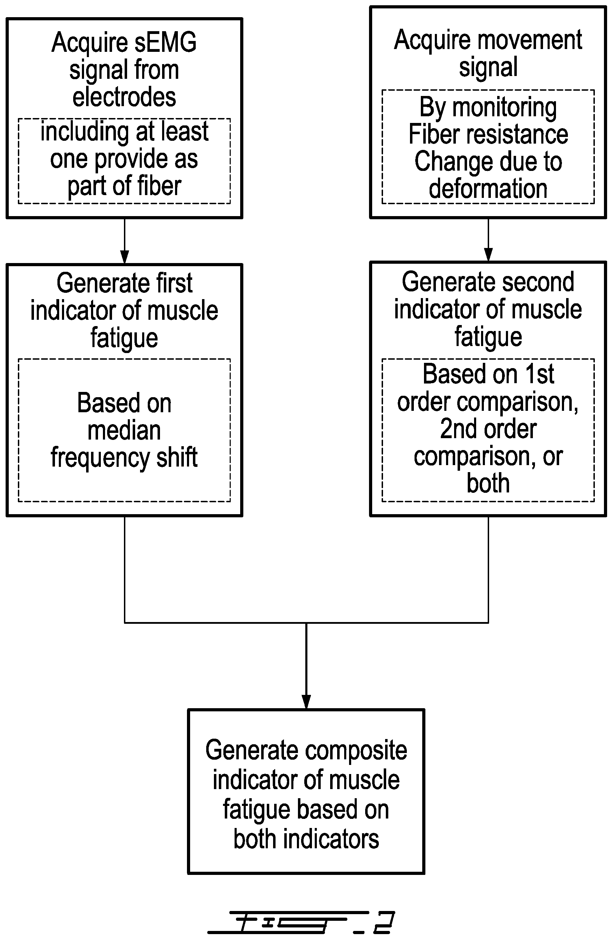

It was found that while such indicators of muscle fatigue can be relevant in and of themselves, their individual limitations could be alleviated to a certain extent by using them in combination to provide a composite indicator of muscle fatigue. This is illustrated in Fig. 2.

Let us now examine each of these potential indications of muscle fatigue individually, before exploring potential ways of acquiring them both and generating such a composite indication of muscle fatigue.

### sEMG

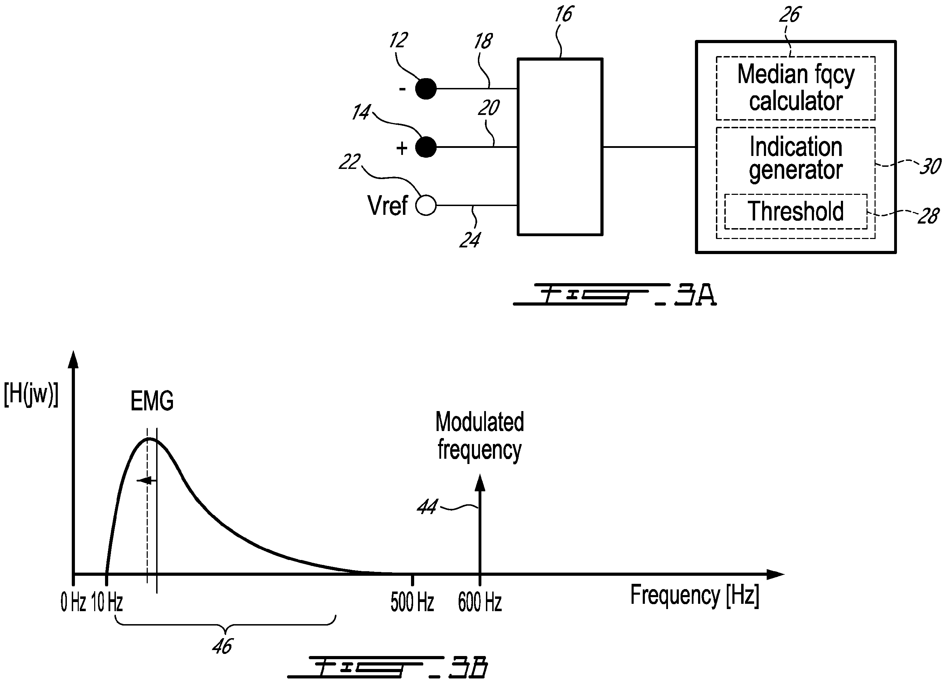

sEMG, to begin, is typically acquired by applying electrodes 12, 14 into contact with the skin covering muscle tissue, such as schematized in Fig. 3A. The sEMG signal is a relatively subtle, low amplitude, electrical signal which causes a change in electric potential between two of the electrodes. This change in electric potential is measured by electronics which we will refer to herein as a sEMG acquirer 16 for simplicity, which is connected to the electrodes by corresponding electrical leads 18, 20. The electrodes 12, 14 can be off the shelf electrodes, for instance. Due to the subtleness of the sEMG signal the sEMG acquirer 16 can require some form of amplification. Moreover, the electric potential of the body can vary over time, and to avoid biasing the measurement of the sEMG signal by the electrical potential of the body, a third electrode 22 is typically used, also connected to the sEMG acquirer 16 by a corresponding electrical lead 24, and driven by the sEMG acquirer to impart a known, reference potential to the body. The electrical leads 18, 20, 24 can connect the sEMG acquirer 16 directly, or via corresponding connectors, for instance. The amplitude of the sEMG signal is typically distributed over a frequency range of 10Hz to 500Hz, such as shown in Fig. 3B, with most of the amplitude being within the range of roughly between 10 and 350Hz, and even 15 and 250Hz, for instance, as shown. The sEMG acquirer can operate over the entire bandwidth of 10-500Hz, but in some embodiments, a suitable signal may be obtained over only one continuous, or discontinuous portion of this bandwidth, especially if it is a portion of the bandwidth which represents the frequencies of highest amplitude of the sEMG signal, for instance.

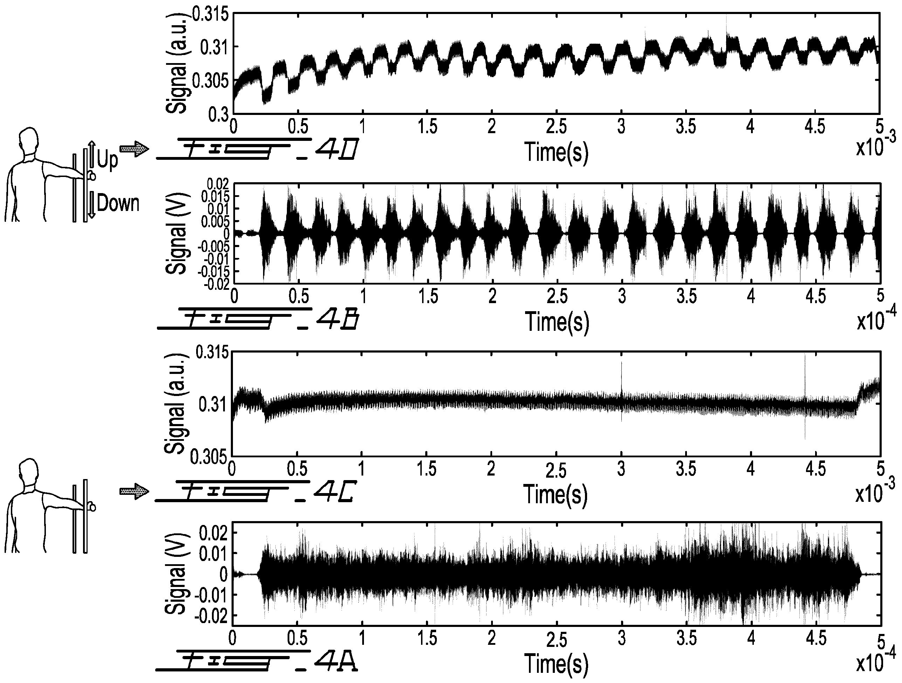

Fig 4A and Fig. 4B present example raw signals which can be acquired in real time by a sEMG acquirer. In this example, the electrodes 12, 14 are on the shoulder of a human user, and Fig. 4A presents a signal acquired during a static posture, while the human user holds a weight statically with his/her hand while the arm is statically held horizontally, and Fig. 4B presents a signal acquired during a dynamic, repeated movement, with the hand and weight moving regularly up and down. By looking at the graphs, one can witness that activation of the muscle entrains sEMG activity.

Returning to Fig. 3A, a median frequency calculator module 26, such as a function operated by a computer, or other electronics, connected in a wired or wireless manner to the sEMG acquirer 16, or otherwise integrated thereto, can be used to determine the median frequency of the sEMG signal. In the static posture presented in Fig. 4A, the median frequency can be repeatedly calculated in real time for instance. In the dynamic, repeated movement scenario of Fig. 4B, software can be used to determine windows of time w1, w2, w3, wn... associated to each one of the repeated movements, and a median frequency of the sEMG signal can be calculated for each one of these windows. A given deviation threshold 28 can be defined, such as by including the corresponding value as data in the memory of the computer for instance, and shifting of the median frequency over time can be measured, and compared to the threshold by an indicator generator module 30, such as a function operated by a computer, or other electronics, connected in a wired or wireless manner to the median frequency calculator module 26, or otherwise integrated thereto. An indication of muscle fatigue can be triggered based on the shifting of the median frequency, such as contingent upon the measured median frequency exceeding the threshold 28, for instance.

Figs. 5A and 5B present an example of the latter process. More specifically, the left hand side 32 of the graph presented in Fig. 5A presents an example of the evolution of the median frequency of the sEMG over time in the absence of fatigue, whereas the right hand side 34 presents an example of the evolution of the median frequency of the sEMG signal over time in the presence of fatigue. A soft threshold 36 is defined in the graph of Fig. 5A in the form of a dashed horizontal line, and a threshold limit 38, or hard threshold, is defined in the graph of Fig. 5A in the form of a continuous horizontal line. The evolution of the indication of muscle fatigue based on this signal on the same timeframe is presented in Fig. 5B. As can be seen in Fig. 5B, the level of the indication of muscle fatigue varies between 0 and 1 as the median frequency varies between the soft threshold 36 and the hard threshold 38, and limits at 1 when the median frequency becomes inferior to the hard threshold (when its shifting exceeds the threshold). Apart from one or two exceptions, it can be seen that the signal indicative of muscle fatigue is generally below 1 for the left hand side 32 of the graph representing the example sEMG without fatigue, and that it is equal to 1 for the right hand side 34 of the graph representing the sample sEMG with fatigue. In one embodiment, the system could generate an alarm when the indication of muscle fatigue remains at 1 for at least a given period of time, such as a time period in the range of a few seconds for instance. However, as will be presented in further detail below, in another embodiment, an alarm can be raised only if (e.g. contingent upon) a composite indication of muscle fatigue based on the indication stemming from the sEMG signal, and at least another indication based on a movement signal, are collectively activated (e.g. at 1) for a given time period, which will be detailed below in reference with Fig. 5G.

The thresholds can be predetermined, or defined in real time. For instance, if one wishes to monitor fatigue by sEMG median frequency monitoring, the threshold can be defined in the form of a shift from an earlier measured value of sEMG median frequency associated to a given user in a given context of use, or from an average of earlier measured values, for instance, and the indication of muscle fatigue can triggered, or increased, when the detected value exceeds the defined shift threshold or otherwise experiences an increasing shift relative to earlier measured values.

Although the signal to noise ratio can likely be improved by selecting more costly electronics, in one example, it was found satisfactory to use a differential mode of measurement using two fiber electrodes and one reference electrode. The two fiber electrodes were positioned in a manner to measure essentially the same biopotential sEMG. In other words, they are positioned on the same muscle. The reference electrode is positioned on a non-articulated portion of the user's body. The difference between the first fiber sensor and the reference electrode is attributed a positive voltage, and the difference between the second fiber sensor and the reference electrode is attributed a negative voltage. Much of the noise (n) generated by the electronics is equally applied to both signals. The second signal, essentially -sEMG +n, is subtracted from the first signal, essentially sEMG + n, yielding 2sEMG, which was found to be a suitable and relatively low cost solution at least in some embodiments.

It is also possible to obtain an indication of muscle fatigue using sEMG on a user subjected to static muscle contraction (i.e. maintained posture). This can be performed, for instance, by measuring the power spectrums and median frequencies of sEMG signals acquired via fiber sensors, for given time intervals (e.g. 1 second). This has been successfully measured on users wearing electrodes on the shoulder muscles, and holding the arm at 90 degrees for as long as they could. The median frequency dropped below the 95% confidence interval of the initial sEMG Power spectrum median frequency, typically before reaching about 50% of the time elapsed between the beginning of the arm holding and the moment where the user considered him/herself incapable of maintaining the requested posture.

### MOVEMENT

Movement can be monitored in various ways, some more elaborate than others. One such way which will be explored in great detail in this specification is by using a sensor having an electrically conductive polymer fiber, designed in a manner for the electrical resistance of the fiber to change when the fiber is bent or otherwise deformed. Other ways of obtaining an indication of movement which can be used in some alternate embodiments can include artificial vision associated with acquisition of the movement within the field of view of a camera, for instance, or a mechanical robotic structure which can be worn by the user and include sensors associated with one or more articulation thereof, configured in a manner for the articulations to be activated, the extent of which is sensed by the sensor(s), when the user exhibits a movement of interest. In at least some embodiments, it was found that the fiber movement sensor referred to above exhibited advantages over the latter examples, for reasons such as ease of use and cost, for instance.

Various materials can be used for the electrically conductive polymer fiber, and a discussion dedicated to this topic will be provided further below.

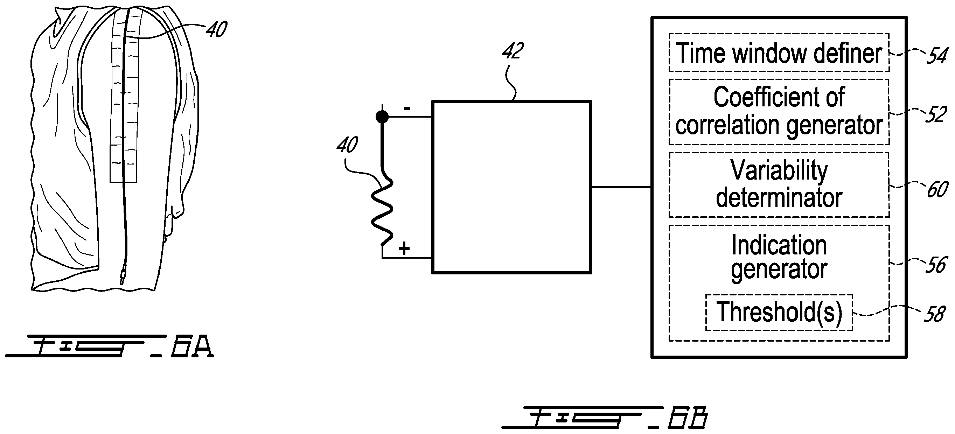

For now, we will simply summarize this technique by referring to Figs 6A and 6B. As shown in Fig. 6A, the technique involves applying the fiber 40 along a given fiber path, ultimately defined relative to the user, as a function of the movement which the technique is intended to monitor. More specifically, the fiber path is selected in a manner to deform upon the relevant movement. In this example, the fiber path extends around the shoulder joint, and along the upper arm of the user, in a manner that it straightens when the arm is moved from the vertical to the horizontal, laterally relative the user. The deformation of the fiber path deforms the fiber which occupies it, which in turn changes the impedence/resistance of the fiber in a detectable manner. It will be noted that Fig. 6A presents a laboratory embodiment where the fiber 40 is held along the fiber path simply by means of an adhesive tape. In commercial applications, the fiber 40 can instead be incorporated to some form of apparel wearable by the user in a predetermined configuration, such that when the apparel is worn by the user in the predetermined configuration, it positions the incorporated fiber along the intended fiber path, within given tolerances. Examples of apparel can include an adhesive patch which is to be adhered to the skin of the user, or an elastic material configured to be stretched upon installation on the user to then be released and hold in place by compression, or secured by fastening means such as buckles or Velcro, for example. More about this will follow.

Turning now to Fig. 6B, the fiber 40 worn by the user forms an interface to the movement, and the change in resistance which occurs upon deformation of the fiber due to the movement, is acquired by electronics which we will refer to herein as a movement acquirer 42 for simplicity. The way the fiber 40 forms an interface to the movement is comparable to the way the electrodes 12, 14 form an interface to the sEMG in the embodiment of Fig. 3A. There are various ways by which the change in electrical resistance can be measured, the simplest being perhaps circulating an electrical current along a significant portion, or the entirety, of the length of the fiber 40, and measuring the changing reaction due to the changing electrical resistance. For instance, if a given voltage is applied by a voltage source, the change in resistance will cause a measurable change in current, and if a given amperage is applied by a current source, the change in resistance will cause a measurable change in voltage. Looking only at the movement acquisition, independently of the sEMG acquisition, one can understand that a DC current can be applied, but it will shortly be demonstrated that in several embodiments, an AC current can be preferred, and can provide an added functionality of allowing simultaneous acquisition of sEMG and movement signal, or better simultaneous acquisition of the sEMG signal, especially when the AC frequency 44 operates outside the sEMG acquisition frequency bandwidth 46, such as exemplified in Fig. 3B.

Fig. 4C and 4D present example raw signals which can be acquired in real time by a movement acquirer 42 using a fiber 40 interface. In this example, the fiber 40 is on the shoulder of a human user, Fig. 4C presents a signal acquired during a static posture, while the human user holds a weight statically with his/her hand while the arm is statically held horizontally, and where we note that over time, when muscle fatigue occurs, the arm begins to lower and thereby cause a measurable change in the movement signal, and Fig. 4D presents a signal acquired during a dynamic, repeated movement, with the hand and weight moving regularly up and down.

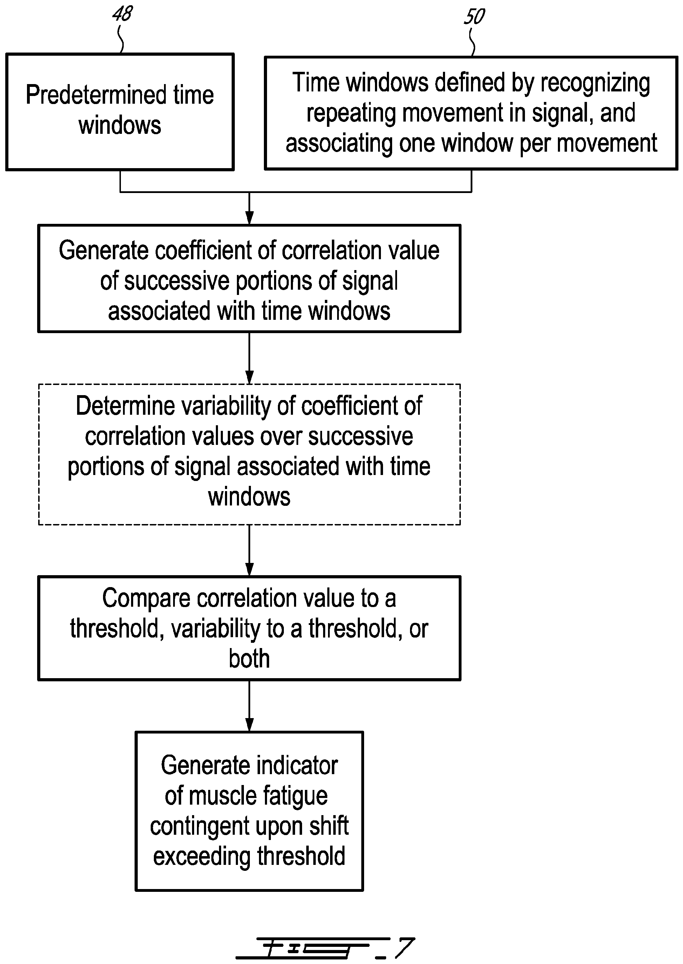

Returning to Fig. 6B, one or more modules or functions provided as part of a computer, or other electronics, connected in a wired or wireless manner to the movement acquirer, or otherwise integrated thereto and to one another, can be used to process the movement signal and to generate an additional, or as we will see in more detail below, possibly two additional indications of muscle fatigue, using a process such as schematized in Fig. 7.

Indeed, two use cases are possible:
the case of a static posture 48 such as exemplified in Fig. 4C, and the case of a dynamic 50, repeated movement, such as exemplified in Fig. 4D, and the example process such as schematized in Fig. 7 can be the same independently of the nature of the movement sensor (i.e. fiber, artificial vision, robotic structure, etc). The example process works on the basis of a comparison of portions of the signal to a given reference, or template, a process referred to herein as "template matching". More specifically, the process compares a portion of the signal within a time window to the template and determines a correlation coefficient indicative of the degree of similitude therebetween. The template can have a given tolerance, such that certain variations can all be considered to be within the tolerance, and in which case the coefficient of correlation can be of 1, for instance, and the more of the signal portion exceeds the tolerance, the closer the coefficient of correlation can be to 0, for instance.

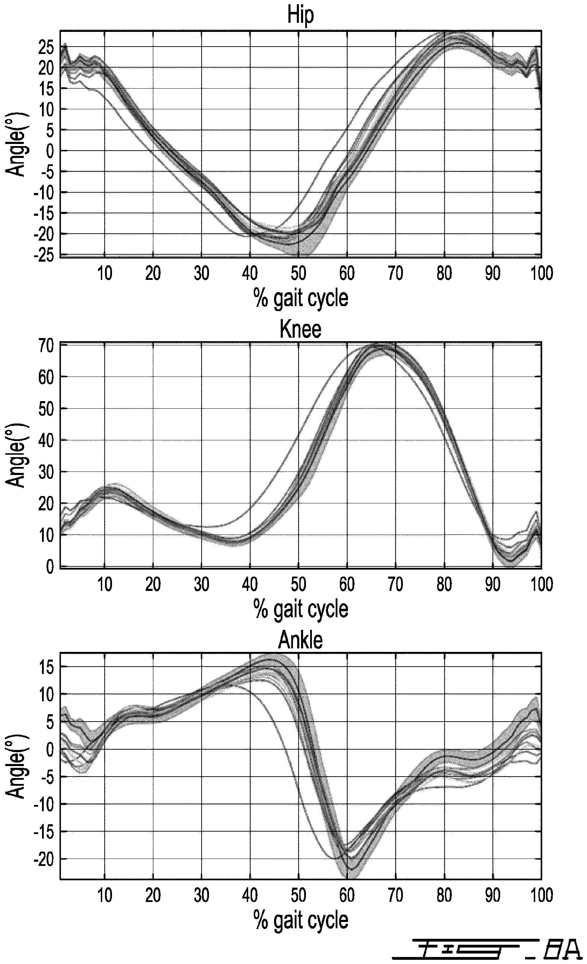

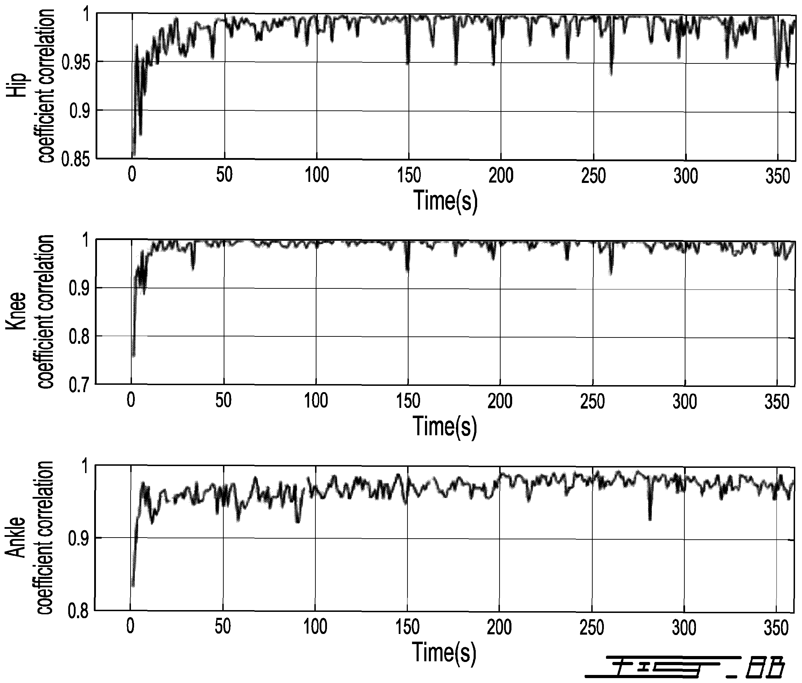

Figs 8A and 8B illustrate the process of template matching in a dynamic context for three movement types, including an ankle movement at the bottom, a knee movement in the middle, and a hip movement at the top. Fig. 8A presents an example of a plurality of individual ones of the windows associated with corresponding ones of the movement superposed on a zone representing the template. The more the individual window matches the template, the higher the correlation coefficient, up to a maximum of 1, or 100% match. The module or function responsible for performing the template matching function can be referred to herein as the coefficient of correlation generator 52.

Indeed, when individuals make repetitive movements such as gait, movement kinematics are stereotyped. While the exact movement features vary across individuals, they can be very repeatable for a given person and form a sort of 'movement signature' (coefficient of variation <=10%). The low variability of movement signatures can be used to detect the gradual development of muscle fatigue using a technique called 'template matching'. First, a reference template (TREF) can be created by averaging a series of initial movement, this can be the first 10-20 movements performed by a user, or to factor in changes in user movement that can occur over time, such as due to a change of weight or a change in muscle force, or long-term learning, it can be reset periodically, such as by taking the first 10-20 movements every day for instance. Considering the low variability of repetitive movements, TREF represents the non-fatigued standard of reference. In the context where it is created each day for each participant, TREF can be highly individually tailored. Muscle fatigue is known to modify movement kinematics, and in particular, to cause a reduction in movement speed and an increase in movement variability.

One difference in which the way the process works between the static and dynamic scenarios is the way the portion of the signal, which will be referred to as the time window, is defined. In the case of a static scenario, the time window can be regularly predefined, such as a sequence of X second time windows for instance (where the number X can be selected in a suitable manner in view of a specific embodiment), and the main focus can be to see whether, for example, a movement is detected over time. In the dynamic scenario, the time windows can be actively defined using a module or function which can be referred to herein as the time window definer 54, which recognizes the presence of a repeated movement, and can then separate the signal timespan into windows w1, w2, w3, wn... corresponding to individual ones of the movements. Two separate indications of muscle fatigue can be obtained from this general process. The module or function responsible for generating the indication(s) of muscle fatigue can be referred to herein as the indication generator 56.

The first indication of muscle fatigue which can be obtained from this process will be referred to as a first order indication, and can simply be based on the amplitude of the correlation coefficient. A given deviation threshold can be defined, such as by including the corresponding threshold value 58 as data in the memory of the computer for instance, and shifting of the correlation coefficient over time can be measured, and compared to the threshold value 58. An indication of muscle fatigue can be triggered contingent upon the shifting of the correlation coefficient in excess of the threshold 58, for instance. This indication can be particularly useful in the static scenario, for instance.

The second indication of muscle fatigue which can be obtained from this process will be referred to as a second order indication, and can be based not on the amplitude of the correlation coefficient, but on the degree of variability of the correlation coefficient over time, which can be an objective indication of an increased wobbliness or clumsiness of the user stemming from muscle fatigue. The module or function responsible for generating the variability of the correlation coefficient can be referred to as the variability determinator 60 herein. A given deviation threshold 58 can be defined, such as by including the corresponding value as data in the memory of the computer for instance, and shifting of the degree of variability over time can be measured, and compared to the threshold. An indication of muscle fatigue can be triggered contingent upon the shifting of the degree of variability in excess of the threshold, for instance. As can be understood, in this specification, the expression "exceeding" is used both to refer to a scenario where a given value is below a given threshold, and where a given value is above a given threshold, for the sake of simplicity.

Figs. 5C and 5D present an example of a process of generating a first order indication of muscle fatigue. More specifically, the left hand side of the graph presented in Fig. 5C presents an example of the evolution of the correlation coefficient of the movement signal over time in the absence of fatigue, whereas the right hand side presents an example of the evolution of the correlation coefficient of the movement signal over time in the presence of fatigue. A soft threshold is defined in the graph of Fig. 5C in the form of a dashed horizontal line, and a threshold limit, or hard threshold, is defined in the graph of Fig. 5C in the form of a continuous horizontal line. The evolution of the indication of muscle fatigue based on this signal on the same timeframe is presented in Fig. 5D. As can be seen in Fig. 5D, the level of the indication of muscle fatigue varies between 0 and 1 as the median frequency varies between the soft threshold and the hard threshold, and limits at 1 when the correlation coefficient becomes inferior to the hard threshold. Apart from one or two exceptions, it can be seen that the signal indicative of muscle fatigue is generally below 1 for the left hand side of the graph representing the example movement signal without fatigue, and that it is equal to 1 for the right hand side of the graph representing the sample movement signal with fatigue. In one embodiment, the system could generate an alarm when the indication of muscle fatigue remains at 1 for at least a given period of time, such as a time period in the range of a few seconds for instance. However, as will be presented in further detail below, in another embodiment, an alarm can be raised only if a composite indication of muscle fatigue based on the first order indication stemming from the movement signal, and at least another indication, are collectively activated (e.g. at 1) for a given time period, which will be detailed below in reference with Fig. 5G, the alarm signaling to the user that he/she should stop the muscular activity and take a rest to avoid injury, for instance.

Figs. 5E and 5F present an example of a process of generating a second order indication of muscle fatigue. More specifically, the left hand side of the graph presented in Fig. 5E presents an example of the evolution of the variability of the correlation coefficient of the movement signal over time in the absence of fatigue, whereas the right hand side presents an example of the evolution of the variability of the correlation coefficient of the movement signal over time in the presence of fatigue. A soft threshold is defined in the graph of Fig. 5E in the form of a dashed horizontal line, and a threshold limit, or hard threshold, is defined in the graph of Fig. 5E in the form of a continuous horizontal line. The evolution of the indication of muscle fatigue based on this signal on the same timeframe is presented in Fig. 5F. As can be seen in Fig. 5F, the level of the indication of muscle fatigue varies between 0 and 1 as the variability of the correlation coefficient varies between the soft threshold and the hard threshold, and limits at 1 when the variability of the correlation coefficient exceeds the hard threshold. Apart from one or two exceptions, it can be seen that the signal indicative of muscle fatigue is generally below 1 for the left hand side of the graph representing the example movement signal without fatigue, and that it is equal to 1 for the right hand side of the graph representing the sample movement signal with fatigue. In one embodiment, the system could generate an alarm when the second order indication of muscle fatigue remains at 1 for at least a given period of time, such as a time period in the range of a few seconds for instance. However, as will be presented in further detail below, in another embodiment, an alarm can be raised only if a composite indication of muscle fatigue based on the second order indication stemming from the movement signal, and at least another indication, are collectively activated (e.g. at 1) for a given time period, which will be detailed below in reference with Fig. 5G.

Fig. 5G presents an example of the process presented in Fig. 2, above, applied to the specific scenario of generating a composite signal of muscle fatigue on the basis of the three available indicators, i.e. 1) the indication of muscle fatigue based on the sEMG median frequency presented in Fig. 5B, 2) the indication of muscle fatigue based on the coefficient of correlation presented in Fig. 5D, and 3) the indication of muscle fatigue based on the variability of the correlation coefficient presented in Fig. 5F. In such a scenario, for instance, an alarm can be generated if and only if two of the three indicators coincide over a given period of time, for instance (e.g. the sum of the indications reaches 2). Alternately, a composite signal can be generated based only on two of these indicators, e.g. the sEMG indicator and the first order movement indicator, or the sEMG indicator and the second order movement indicator, for instance. In still another example, the same, or different weight, can be given to two or three of the individual indications of muscle fatigue, in the form of a multiplier factor for instance, to form the composite indication of muscle fatigue, and an alarm can be triggered when the composite indication of muscle fatigue value reaches or exceeds a given threshold, for instance.

The thresholds can be predetermined, or defined in real time. For instance, if one wishes to monitor fatigue by movement detection, the threshold can be defined in the form of a shift from an earlier measured value, or from an average of earlier measured values (such as from a number of earlier dynamic movements or from an earlier period of static posture), for instance, and the indication of muscle fatigue can triggered when the detected value exceeds the defined shift threshold. In another example, the threshold can be set in a fixed predetermined manner, such as by setting the threshold as a given value of correlation coefficient under which the indication of muscle fatigue is triggered, for instance.

### COMBINATION OF MOVEMENT AND sEMG

In order to be able to alert a user in real time, the indicators need to be generated relatively simultaneously, meaning within a timeframe which is small compared to the timeframe within which an alarm should be triggered if we are to alert the user in advance of an undesired consequence such as an injury. There are specific challenges in achieving this while also providing a suitable degree of precision in the indication and a device of a sufficiently low cost to make it commercially viable/appealing.

One approach can be to provide a sensor which includes the sEMG electrodes and the sEMG acquirer separately from the movement sensor and the movement acquirer. However, this requires independent hardware and does not benefit from any potential hardware sharing economy, and may not be considered commercially viable/appealing in all embodiments.

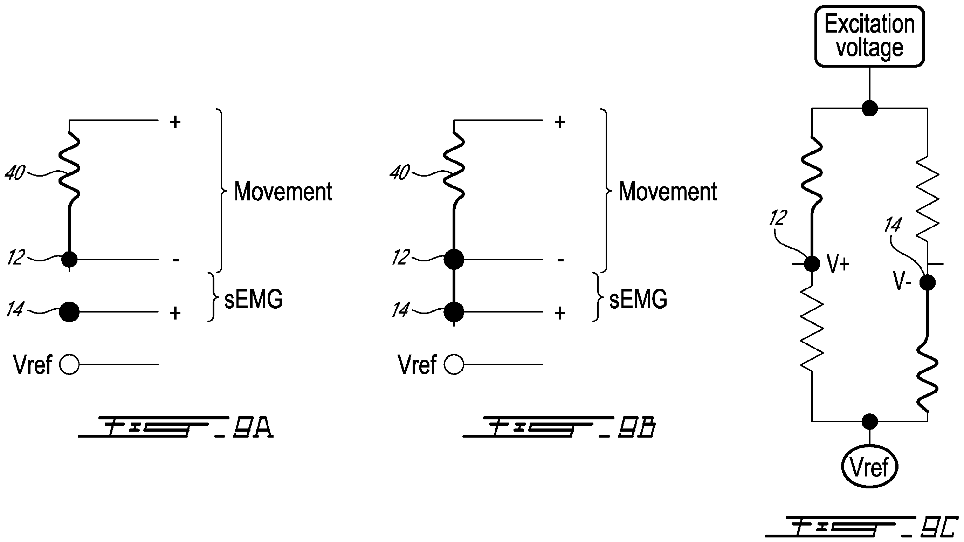

Another approach, which is the one implemented by the claimed method, can be to share hardware between the subsystems. For instance, the fiber 40 is electrically conductive, and could therefore serve as the basis for using it as one electrode 12, such as schematized in Fig. 9A, or even as two electrodes 12, 14 such as schematized in Fig. 9B. As will be seen further below, in some embodiments, an example of which is schematized in Fig. 9C, it can be preferred to use two fibers, in which case each one of the fibers can have one of the electrodes 12, 14. To use a portion of the length of the fiber as an electrode, that portion is to be maintained into direct contact with the skin during use, similarly as to how an off-the-shelf electrode would be maintained into direct contact with the skin. As discussed above, to this end, the apparel 62, 64 to which the fiber is incorporated can either be configured to adhere to the skin in the area adjacent the electrode portion of the fiber, or be configured to compress the electrode portion of the fiber against the skin due to its elastic nature, or due to the presence of some form of fastening or attachment, to name two examples.

However, in such scenarios, the electrical drive of the movement acquirer can generate electricity within the user's skin which can interfere with the subtle sEMG signal which the sEMG acquirer 16 is designed to acquire. This may also be the case in scenarios where the sEMG acquirer 16 and electrodes 12, 14 are provided entirely independently from the fiber and movement acquirer 42 from the electrical point of view, especially when they are used in proximity with each other, which can be required for obtaining two independent indicators associated to a localized muscle or joint.

To avoid this interference, it can be required to multiplex the signals in one way or another. One way to multiplex the signals is to multiplex over time. In other words, to acquire the sEMG signal and the movement signal at alternating moments in time. There can be some complexities to achieve this without a dissuasive amount of noise from the hardware perspective, and an additional inconvenience is that since the sEMG and the movement signals are not achieved simultaneously, this can cause a delay in the generation of an eventual alarm destined to the user, which may be undesired.

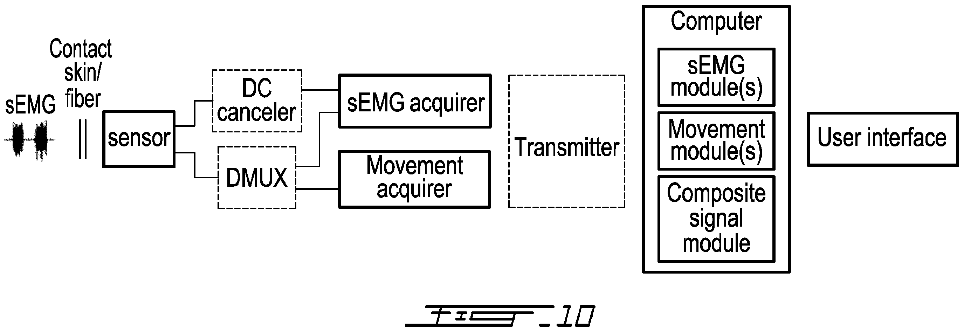

One way to allow the acquisition of the sEMG and movement signals simultaneously is to multiplex the signals over the frequency domain. An example of this is schematized in Fig. 3B where the drive frequency 44 of the movement signal acquirer is defined to be 600Hz, which can be well outside the frequencies of interest of the sEMG signal 46, for instance. In other embodiments, it can be preferred to chose a drive frequency of 800Hz, or of 1kHz, or more, for instance. At such frequencies, capacitors can act essentially as short circuits, while canceling and DC component. In this manner, filters can be used to demultiplex the movement signal bandwidth from the sEMG signal bandwidth, and more about this will be detailed further below. It will also be noted that the AC drive of the movement acquirer can be expected to have a DC component which can need to be canceled from the point of view of the fiber. This can be achieved by using an DC canceler, which can involve an AC coupling, such as capacitors, between the fiber and the electronics. The details of the AC coupling can vary from one embodiment to another, examples of which will be presented below. For now, let us consider an example system which can use one or two fibers, and three electrodes, one or some of which can be provided as corresponding portions of the length of the one or two fibers, a SEMG acquirer, a movement acquirer, and some form of multiplexing and/or demultiplexing, together with DC cancellation, such as schematized in Fig. 10. Such a system can be connected to a computer, either wiredly or wirelessly, and the computer (e.g. PC, cellphone, other data processing machine) can have some form of user interface, which can convey an alarm of muscle fatigue based on a composite indication of muscle fatigue to a user, for example.

Example ways to embody such electronics of the system will be presented further below, but first, we will look into the question of the interface into further detail.

### Fiber Material

Various polymer materials can represent suitable properties of being electrically conductive while having a resistance which changes when it is deformed. The exact choice thereof can be left to the designer of a specific embodiment, and can be affected by the exact embodiment. One may wish, for instance, to strike a suitable balance between conductivity, flexibility and durability.

In many embodiments, the fiber should be suitably long, i.e. more than 10 cm, more than 15 cm, and many applications will benefit from a length of fiber of between 20 and 40 cm. The process of making such a fiber can vary from one application to another, and the specifics of two example fibers will be presented here for the purpose of providing illustrative examples from which a designer can inspire him/herself in the production of a suitable fiber in view of a specific embodiment.

In one example embodiment, tests were conducted on the basis of a matrix of ethylene and vinyl -acetate, more specifically *Poly(ethylene-co-vinyl acetate)* - (PEVA). This choice was made on the basis that it was a thermoplastic polymer easily accessible on the market and having a relatively low cost, and being relatively easy to use. Carbon nanotubes and graphite are examples of conductive materials which can be incorporated into the polymer matrix to give electrical conductivity to the fiber in a relatively precisely predefined concentration. In this embodiment, it was determined that current would likely pass more easily along the fibers of the nanotubes than across the layers of graphite fibers, and therefore carbon nanotubes were selected. In the tested example, >98% purity, multi-walled carbon nanotubes (MWCNTs) were selected as the conductive material.

10 different mixes were tested with different concentrations of carbon nanotubes in a PEVA polymer matrix. More specifically, concentrations of 10% by weight of carbon nanotubes to 100% by weight of carbon nanotubes (1g carbon for 1g PEVA) were tested, produced using cold syringe extrusion. The most interesting results were in the range of weights of carbon nanotubes having 50-80% of the weight of EVA. It is believed that slightly more and slightly less could also be suitable. Weight ratio around 60% appeared ideal in this embodiment. At higher carbon nanotube weight ratios, the fibers became so brittle that there were less interesting from the point of view of repeated uses, especially if intended for use in clothing where washing processes can generate significant stress on the fibers. At lower carbon nanotube weight ratios, the fiber sensor created more resistance and was less well adapted to reading sEMG signals. Resistance values between 8 and 10 Ohms/cm were considered interesting. In the case of MWCNTs and PEVA, it can be preferred to perform the sonication in tetrahydrofuran for instance, and to dry the resultant colloidal solution in an oven at 100 °C for 15 min to obtain a composite with high viscosity.

Various forms of testing were conducted on such fibers, and following this testing, there remained good candidates for use in wearable technology incorporated to an elastically stretchable apparel. Indeed, they were exposed to Palmolive^{™} (pH 7) detergent for an equivalent of over 25 washing cycles. Although the test fiber sensors loss on average about 5% of their conductivity following the washes, this was considered satisfactory. Similar tests were made in a washing machine with Tide Advanced Power detergent. This did not cause a significant reduction in conductivity, and the fiber sensors were thus considered resistant to washing machines. Resistance to sweat was also tested. 11 samples of 2 cm each were tested with 1.5 mL of saline solution for different durations ranging from 3 minutes to 5 hours. This did not lead to any significant degradation of the electrical conductivity. The material was also tested with urea in a concentration of about 27 grams / 25 mL of water. In cases where the urea solution was allowed to dry, a loss of conductivity was detected, however this loss of conductivity was reversed after washing with a Tide detergent, and therefore it was concluded that the fiber sensors with this combination of materials was resistant to sweat for even after 6 hours exposure.

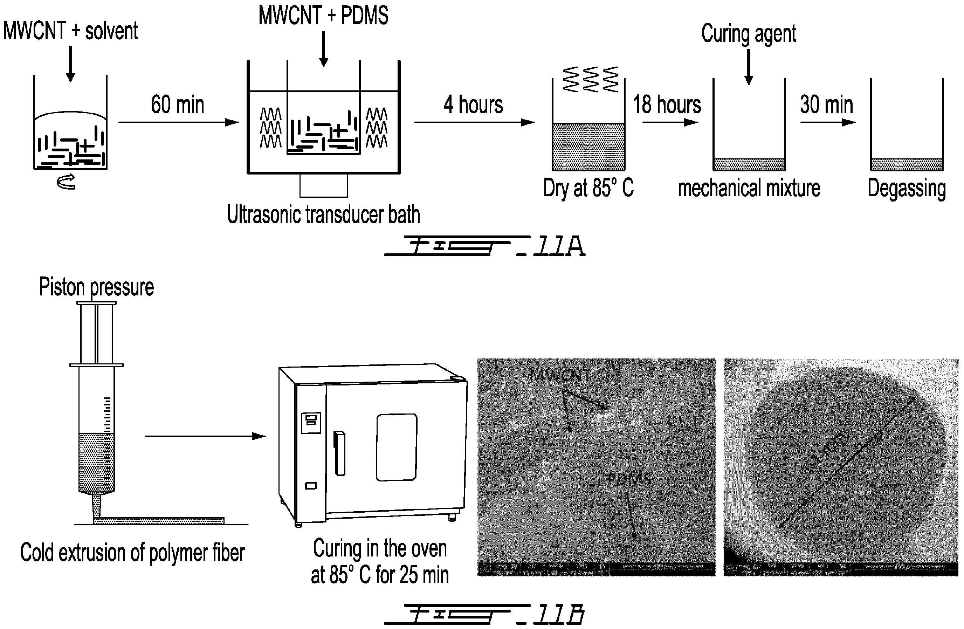

Fig. 11A schematizes a fabrication process of an alternate example of conductive flexible polymer using MWCNT and polydimethylsiloxane elastomer (PDMS). The mixing process was carried out as follows. To favor dispersion of the conductive nanoparticles, the MWCNT nanoparticles were sonicated for 60 min in iso-propanol solvent (Fisher Scientific International, USA) with a ratio of 1:100 by mechanical stirring for 1h at 1400rpm. Then, the base of the Sylgard^{™} 184 silicone elastomer kit (PDMS -A) was blended with the MWCNTs solution and sonicated for 4 hours to ensure a better penetration of the nano-filler inside PDMS-A. The resultant colloidal solution was dried in the oven at 85 C for 18 hours to obtain a composite with high viscosity. The cross-linker PDMS-B was added to the composite and vigorously mixed for 30 min. A vacuum desiccator was used to remove the bubbles remaining from the mixing process.

A conductive polymer fiber with customized length and diameter was prepared using the resulting material by an extrusion technique using a syringe mounted on an extrusion machine. After loading the syringe with the prepared composite, continuous fibers were extruded on a low-energy surface sheet then cured in an oven for 25 min at 85 °C. Fig. 11B shows an SEM cross section of MWCT/PDMS fiber of 1.1 mm diameter.

Typically, increasing the % mass of carbon relative to the % mass of flexible polymer will result in an increase in electrical conductivity (i.e. lower resistance) at the tradeoff of a loss of flexibility. The exact choice may be best made as a function of the details of a specific application, such as taking into consideration the exact area where the fiber electrode sensor is intended to be used on the body of the mammal. In applications where both flexibility and electrical conductivity are relevant factors, and depending on the exact choice of polymer used, and in the particular case of a PDMS matrix, compositions having between 5wt% and 7 wt% of carbon can be of particular interest and offer an interesting balance between flexibility and conductivity.

### Fiber path and connection configurations

It was found that somewhat different considerations existed whether one takes the point of view of optimizing the fiber, and system, for sEMG acquisition or the point of view of optimizing the fiber, and system, for movement acquisition. Let us now explore these considerations and then the question of striking a balance between the two.

Indeed, in general, when taking the point of view of sEMG acquisition, greater electrical conductivity can be preferred, and the quality of the electrical contact between the fiber and the skin is highly relevant as it affects the quality of the electrical "connection" between the fiber and the signal. It was found, in particular, that when using a portion of the length of a fiber as an electrode, better signal quality was obtained when the electrode portion of the fiber was oriented transversally relative to the orientation of the muscle fibers. Moreover, the sEMG signal having a low amplitude, sensitivity of acquisition can be a particular concern. Amplification and cancellation of noise can be particularly useful, depending on the embodiment.

When taking the point of view of movement acquisition, the extent of resistance change upon deformation can be one feature that one may wish to increase to the extent possible, to provide a greater sensitivity to the movement acquisition. Moreover, the portion, or entirety, of the length of the fiber which serves as a basis for movement acquisition does not need to be in good electrical contact with the user's skin, it only needs to occupy a fiber path defined relatively to the wearer and which reliably deforms when the target muscle is activated, or when the target movement otherwise occurs. When effectively multiplexed relative the sEMG frequencies and DC-shielded from the electrodes, the amplitude of the movement signal can, in some embodiments, be increased by raising the amplitude of the AC drive, which can, to a certain extent, make questions of sensitivity easier to resolve.

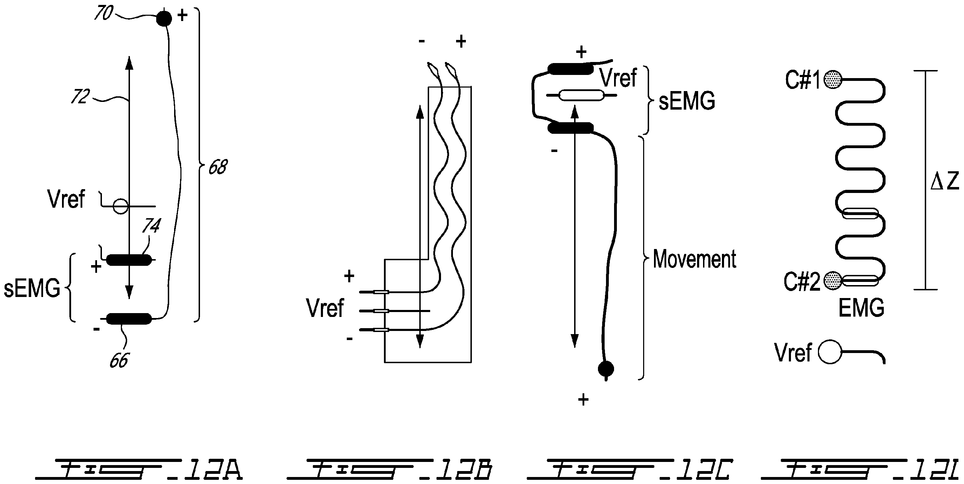

Fig. 12A is one example of fiber path and connection configuration. In Fig. 12A, a single fiber is used as a basis of the movement acquisition, and this fiber has an electrode portion 66 maintained in direct electrical contact with the wearer's skin, and a portion 68 of its length dedicated to movement acquisition extending between the electrode portion 66 and the connection to the other electrical lead 70 serving as a basis for movement acquisition. The latter portion 68 of the length will be referred to as the movement portion 68, and can be seen to form a relatively linear shape having a length extending generally in the orientation 72 of the muscle fibers. The electrode portion 66 also has a certain length, and the length of the electrode portion 66 generally extends in the orientation transverse to the orientation 72 of the muscle fibers. A second electrode 74 used for the purpose of sEMG acquisition is provided here as a significantly shorter fiber which is dedicated solely to sEMG acquisition, and therefore does not have any electrical lead leading to the movement acquirer. Still a third electrode Vref can be used, which is provided here in the form of another short fiber for convenience, to impose a reference voltage to the body of the wearer, relative to which the sEMG signal is acquired.

Fig. 12B shows another example of a fiber path and connection configuration. Fig. 12B bears similarities to the embodiment presented in Fig. 12A, with a few differences. For instance, two fibers having movement portions are used instead of one, and it will be seen in greater detail below that, particularly when the resistance of both these movement fiber portions is intrinsically balanced, and when their path is configured in a way such that they deform similarly upon the movement, such dual movement fiber portion configurations can be useful because the movement acquisition can then based on a differential measurement which can then both amplify the signal and cancel out noise. Moreover, the shape of the movement portion of the fibers here is, although generally elongated in the orientation of the muscle fibers, somewhat transversally sinuous. It was found that introducing sensuosities into the fiber path of the movement portion of the fiber(s) can be beneficial in some embodiments, as it can make the fiber(s) sensitive to torsion in the transversal orientation, in addition to being sensitive to bending of the muscle fibers. Moreover, the sEMG acquisition electrodes are embodied here as respective electrode portions of respective ones of the long fibers. The reference electrode is also provided as a fiber, a short one in this case, for convenience, which is oriented transversally, parallel to the electrode portions, and between the electrode portions, separated from each electrode portion by a similar distance.

Fig. 12C shows another example of a fiber path and connection configuration bearing similarities with the embodiment of Fig. 12A. In this example, the main difference is that both sEMG electrodes are embodied as distinct electrode portions of the length of a single fiber. To this end, the single fiber has a transversally oriented U shape at one end, both branches of which serving as a corresponding, transversally oriented, electrode portion.

Fig. 12D is another example of a fiber path and connection configuration bearing similarities with the embodiment of Fig. 12C. The main difference being that the movement portion of the length of the fiber has additional sinuosities which may be useful in this embodiment to sense torsional components of the movement transverse to the orientation of the muscle fibers, in addition to bending of the muscle fibers.

The notion of defining a fiber path and electrode configuration relative to the wearer may appear, at first glance, somewhat abstract, when looking at the sensor when it is not being worn by the wearer, but we will see that in practice, it is not abstract at all. Indeed, although there can be many uses for such a sensor, some of which may be in medical environments, others of which may be in sports environments, and that specific uses can target different joints or movements of the wearer, such uses will typically be predetermined from the point of view of designing the particular sensor.

In other words, different sensors will be designed specifically for corresponding uses, and the fiber path will be defined relative to the corresponding apparel, and the corresponding apparel will be specifically designed to be worn in a manner associated to the specific use.

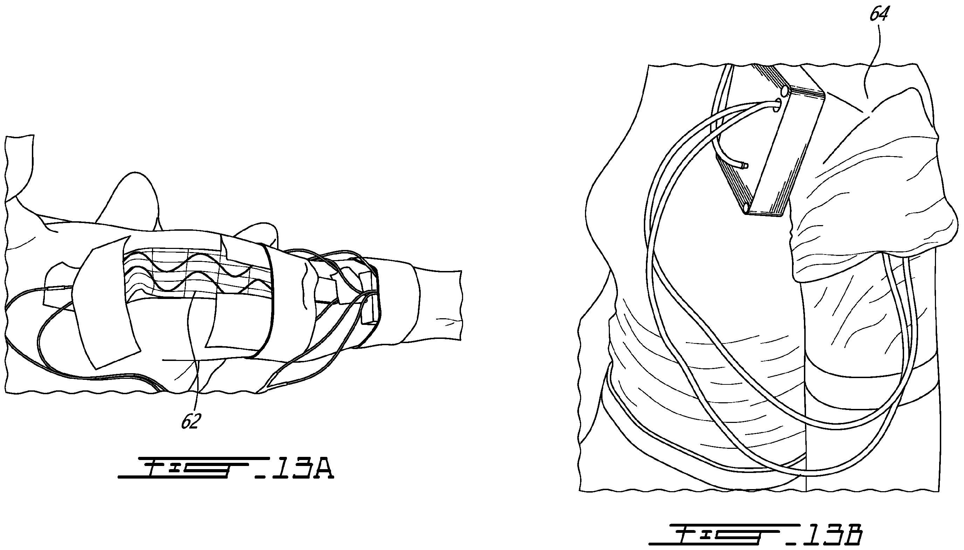

For instance, in Fig. 12B, the apparel is an adhesive textile element, which can be manufactured with the fibers incorporated to the adhesive textile element by adhering them to it in accordance with specific fiber paths. The adhesive textile element can be provided with a paper backing configured to be removed upon use, to expose the fibers and the adjacent adhesive face, and allow its application on the specific part of the wearer's body which it is intended to cover. In the specific case of the example presented in Fig. 12B, this part of the wearer's body is the shoulder and upper arm portion, as can be seen from the picture presented in Fig. 13A, which shows the sensor worn by the wearer and ready for use.

If the sensor is designed to cover the hip of the wearer, or the lower back, or the ankle, to name some possible alternate examples, the size and shape of the apparel, the size and shape of the fiber(s), and the path occupied by the fiber(s) when it is incorporated to the apparel, will be adapted specifically to the target movement/use. Generally, this can involve orienting the major dimension of the movement portion of the fiber along the length of the associated muscle fibers, and can also involve orienting the length of the electrode portion(s) of the fiber(s) transversally to the length of the associated muscle fibers to favor sEMG signal quality.

Adhesive textile element apparel types may be well suited for single-use applications, which may more frequently occur, for instance, in medical situations. In other situations, it may be preferred to design the sensor in a manner to be re-usable, at least to a certain extent, which can more frequently occur, for instance, in consumer or athlete sport situations. One type of apparel which may be more suitable for re-usable applications can be apparels provided in the form of garments made of elastic material and designed to be stretched upon mounting to the corresponding body part, and then released to hold in place simply by compression. In still other situations, it can be preferred to use apparel which includes garments having limited elasticity, but which can be affixed onto the body parts with fastener elements such as Velcro. Fig. 13B presents an example of a sensor which includes one or more fibers incorporated into an apparel provided here in the form of garment, or band, worn on the wearer's shoulder and upper arm.

Independently of the specific form of apparel selected to form the support to the fiber(s), the apparel can have the function of holding the fiber(s) in a predetermined fiber path relative to the wearer for movement acquisition, and holding the electrodes into electrical contact with the wearer's skin for sEMG acquisition.

### EXAMPLE EMBODIMENTS OF SYSTEM FUNCTIONALITIES

Fig. 10 provides one example system which can use a sensor having, supported by an apparel in a functional manner, one or more fibers, and three or more electrodes, one or more of which can be provided in the form of corresponding electrode portions of the length of the one or more fibers, such as explained above.

Such a system can include a group of elements which can be referred to collectively as a sEMG acquirer, and whose functionality can be summarized as to extract an electronically useable sEMG signal from the sensor. Such a system can also include a group of elements which can be referred to collectively as a movement acquirer, and whose functionality can be summarized as to extract an electronically useable movement signal from the sensor. In one embodiment, sEMG acquirer can include an amplifier, an analog to digital converter, and means to set the Vref voltage in the reference electrode, though it will be understood that in some alternate embodiments, one or more of these components can be omitted or substituted by something else. In one embodiment, the movement acquirer can include an AC electrical drive, an amplifier and an analog to digital converter, though it will be understood that in some alternate embodiments, one or more of these components can be omitted or substituted by something else. In some embodiments, one or more of the elements can be shared between the sEMG acquirer and the movement acquirer to achieve hardware economy.

Such a system can also include one or more elements which can be referred to collectively as a demultiplexer, and which can include one or more filters, for instance, to direct corresponding frequencies to the sEMG acquirer and to the movement acquirer, such as in a scenario where the AC electrical drive operates at a frequency which is outside the sEMG bandwidth of interest. The filters can also be used to filter out other sources of noise if needed. Amplification can take place between the sensor and the demultiplexer in some alternate embodiments.

Such a system can also include one or more elements which can be referred to collectively as a DC canceler. Indeed, AC electrical drives can typically be expected to involve a DC component, and it can thus be desired to cancel out such a DC component before the AC electrical drive signal reaches the sensor, to avoid interfering with the sEMG acquisition. The DC canceler can be provided in the form of one or more capacitors for instance.

The functions presented above in association with the sEMG acquirer, the movement acquirer, the demultiplexer, and the DC canceler can be considered to form basic functionalities which, in some embodiments, it can be preferred to embody as electronics, examples of which will be presented further below.

The example system presented in Fig. 10 presents a scenario where higher functionalities are performed by a computer. Such higher functionalities can include sEMG modules or functions such as a median frequency calculator, a sEMG median frequency comparator, and a sEMG muscle fatigue indication generator. Such higher functionalities can also include movement modules or functions, such as a template matching coefficient generator, a coefficient comparator, a first order muscle fatigue indication generator, a variability determinator, a variability comparator, and a second order muscle fatigue indication generator. Some of modules or functions can be shared between sEMG and movement. For instance, in an embodiment adapted for dynamic repeated movement scenarios, such higher functionalities can also include module or function which operates to recognize a pattern of repeated movement, and separates the signal in time windows associated to individual ones of the movements of the pattern, and which can be referred to as a window determinator, for instance. Such higher functionalities can also include a composite muscle fatigue indicator generator configured to generate a composite indication of muscle fatigue based on two or more of the individual indications of muscle fatigue referred to above.

The example system can also include a user interface configured to allow interaction with the user. In a simple example case, where the computer is in also incorporated to the apparel or otherwise worn by the user, for instance, the user interface can be a visual or audible alarm which can be triggered based on the composite indication of muscle fatigue. In a more elaborate example case, the computer can be provided remotely, and signals or associated data can be communicated to the computer in any suitable way, such as via the Internet for instance, and the computer can be provided with elaborate software allowing more in depth analysis, for instance.

In all embodiments, the sensor will be worn by a wearer during the process of signal acquisition. It some embodiments, the sEMG acquirer, the demultiplexer, and the movement acquirer can be housed in a preferably relatively small, one or more, electronics housing(s) which can also be worn by the user. In some embodiments, for instance, it can be preferred to provide the system with connectors, to allow to easily connect and disconnect the electronics housing from the electrical leads of the sensor. This can be useful, for instance, if the sensor is embodied as a disposable component, for instance, in which case the electronics housing can be re-used with another sensor. Such a scenario can be particularly useful in medical applications, for instance, where the sensor is provided with a single-use adhesive apparel, for instance, but can also be useful in sports applications where the sensor can be expected to begin to wear and potentially malfunction after a certain number of uses or washes, or simply to allow removing the electronics housing before washing the sensor, and reconnect it afterwards. In such embodiments, it can be preferred to either incorporate the DC canceler to the sensor, or within the electronics housings, and the ultimate choice can be left to the designer in view of the specifics of corresponding embodiments.

Some, or all, of the aforementioned higher functionalities can also be integrated into such one or more electronics housing(s) configured to be supported by the apparel, or by a separate computer, and the exact choice of which can be left to the designer in view of the specifics of corresponding embodiments. For instance, in some embodiments, a transmitter can be integrated to an electronics housing supported by the apparel, and configured to transmit relevant data to a computer. In a relatively simple example, the transmitter can transmit the raw signals of the acquirers, or processed data if one or more higher functionalities are incorporated to the electronics housing, to a smartphone, smartwatch, or other computer, worn or held separately from the apparel by the user. Such a smartphone or smartwatch can have a software application configured to perform one or more of the higher functionalities and interact with the user via the user interface, for instance, and can further be configured to communicate some relevant data to another computer over the Internet or other network. In a more elaborate example, the transmitter can transmit the raw signals of the acquirers, or processed data if one or more higher functionalities are incorporated to the apparel, directly over the Internet, for access by a healthcare professional or trainer, for instance. The transmitter can be controlled by a microcontroller (MCU) which receives the signals from the sEMG acquirer and the movement acquirer, for instance.

As will be understood from the above, various scenarios and embodiments are possible, and specific embodiments can be adapted or optimized in view of the specific scenarios considered.

### EXAMPLES EMBODIMENTS OF ELECTRONICS MODULES

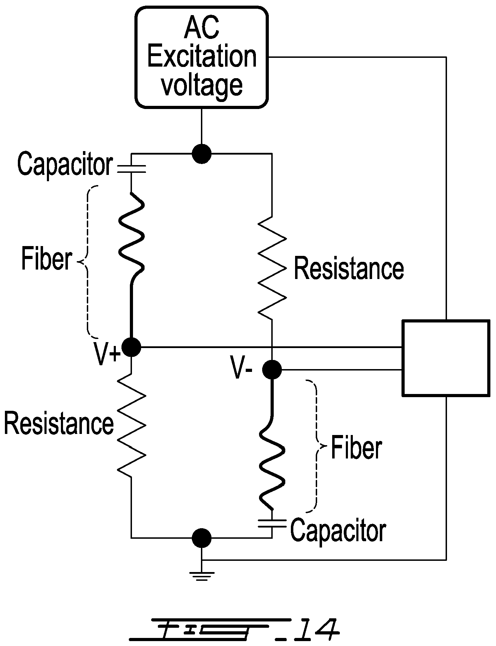

Fig. 14 presents a first example embodiment. In this embodiment, two fibers are used, in a "half-full Wheatstone bridge" configuration, with two other resistances. The AC electrical drive applies an AC tension at a frequency of 790 Hz, which is somewhat higher than the maximum sEMG frequency, opposite a reference electrode which is connected to the right leg driver of the circuit. The sEMG acquirer and the movement acquirer are somewhat combined, as the tension measured between the V+ and V- points is the sum of the movement signal, at 790Hz, and of the sEMG, between 10 and 500Hz. Capacitors are used as a DC canceler, to ensure a virtually infinite DC impedance between the measuring points of the sEMG, to avoid flooding the sEMG by imposing a DC tension on the body, and entrain a high DC impedence between the two measuring points. The movement portion of the fibers is electrically insulated from the skin, with only the electrode portion of the fibers being in intimate electrical contact with the skin. An analog antialiasing fiber of 10Hz-900Hz can connect the signal to an analog/digital converter, via an amplifier if needed. In one example embodiment, the sum of the signals can be communicated in its relatively raw, though digital, form to a computer, which can be achieved by wireless transmission for instance, or by storing the data on a removable medium, and then transferring the removable medium to a reader associated with a computer. The sEMG signal can be isolated by a Thebychev Type II pass-band filter between 10 and 500Hz. A spectrograph having 1 second windows can be applied to obtain the frequency content allowing to calculate the median frequency. For the movement signal, a passband signal between 780 and 800Hz can be applied to isolate the multiplexed frequency. A 100 factor interpolation can be applied to counter the beating effect.

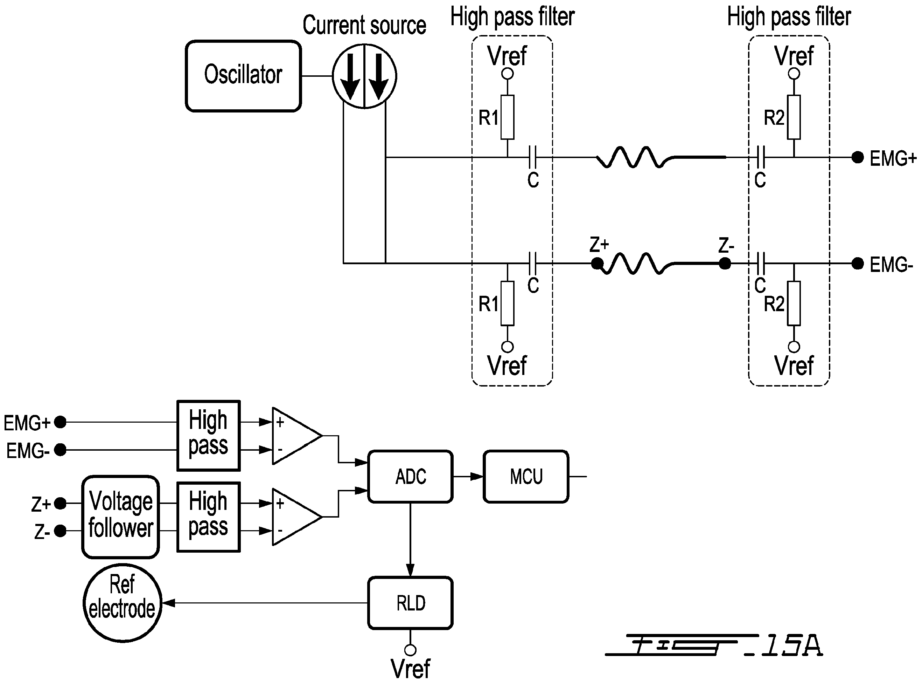

Fig. 15A presents a second example embodiment. This embodiment as well uses two fibers. The operation consists essentially in injecting the AC drive into the fibers in a manner that it will be modulated in amplitude by the variation in impedance. The sEMG is taken as a differential between the two fibers. To achieve a satisfactory degree of precision, it may be required for the two fibers to be virtually identical. AC current generated by an oscillator is injected in an equal manner in the two fibers with current sources. The modulated signal is measured at points opposite a movement length of one of the fibers, whereas the sEMG is measured between the two fibers. If the injected current is identical, and the two fibers are identical, the sEMG can be acquired via a differential amplifier between the two fibers. As can be seen, a DC canceller is provided here in the form of capacitors provided in the electrical path of each fiber, including one before the fiber and one after the fiber.

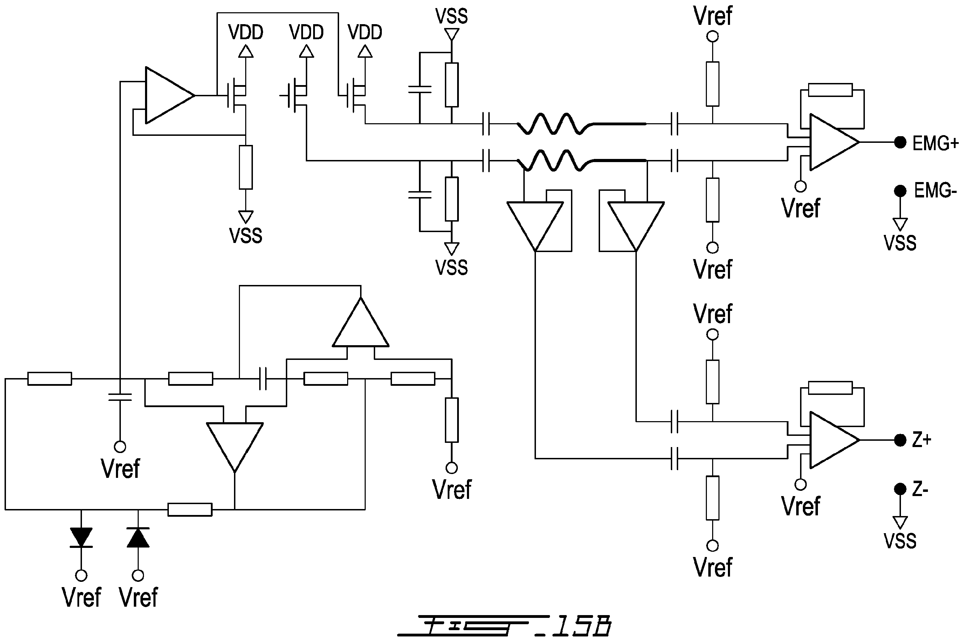

Fig. 15B is a detailed schematic of an example embodiment in accordance with the diagram of Fig. 15A. A current mirror injects the same current in both fibers which allows the injected noise to be rejected as a common mode in the sEMG measurement. This can allow to significantly increase the gain of the instrumentation amplifier without saturation. High pass filters are used at the exit of the current sources are used, with a cutoff at 490Hz to allow the AC signal. A high pass filber having a cutoff at 500Hz can be positioned before the instrumentation amplifier to cancel the DC and the sEMG frequency bandwidth. Tension followers can be used at the points on the fiber o avoid interference between subsystems.

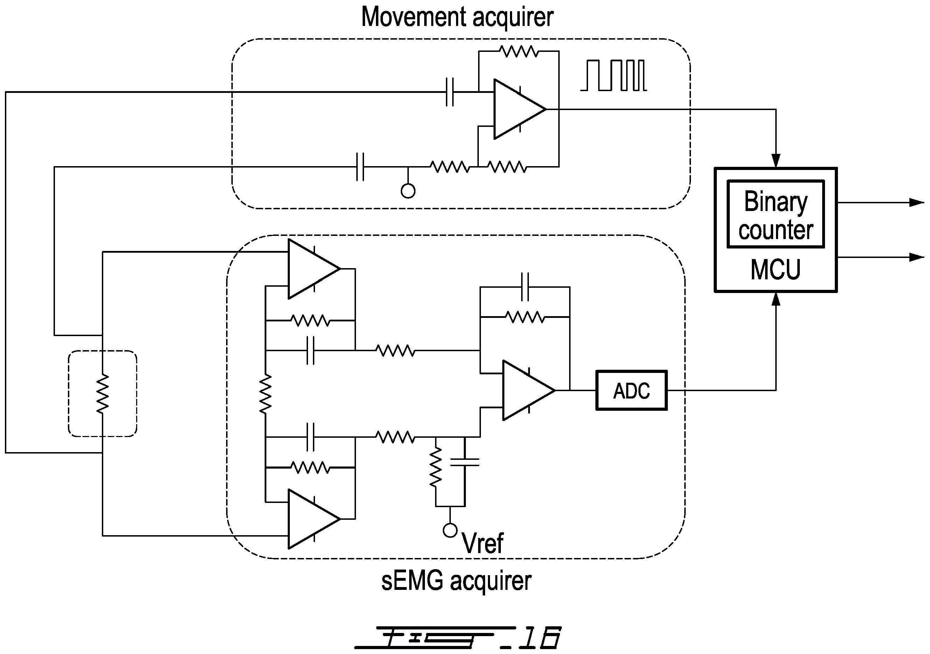

Fig. 16 presents a third example embodiment. This embodiment uses a single fiber in a configuration such as shown in Fig. 12D. Such an embodiment can be robust to imperfections and variability of the fiber. In this scenario, the change of impedance is encoded as a shift in frequency, as the AC electrical drive forms an oscillator with the changing resistance of the fiber. An instrumentation amplifier with capacitors allows to amplify only the frequencies of the EMG extending between 10 and 500 Hz, and to filter/reject the drive frequency, which can be of 600Hz here for instance. In this specific embodiment, the change of frequency is measured with a counter counting the zeroes of the oscillation, which allows to derive the change of impedance of the fiber. The drive frequency is configured to remain well outside the sEMG spectrum even in the case of a maximum deformation of the fiber associated to the targeted movement. In this specific embodiment, the DC canceler is embodied as capacitors integrated to the movement acquirer.

Independently to being used in relation with providing an indication of risk of injury, a system such as presented herein can alternately be provided to assist athletes in training, for instance, such as by providing guidance for technique correction for instance. For example, a volleyball service requires a great degree of precision and synchronization on the sequence of execution: throw, run, jump, strike, to have an impact point as high as possible. Using a system such as presented above on one or more muscles could help guiding the athlete in terms of improving the timing of his movements. It could also allow comparison of different high level athletes to one another. Other similar applications could occur in many high level sports, such as swimming, basketball, football, etc.

Another example of an alternate application is to provide indication on the consistency of movement of an athlete to a coach during a game. Such an objective indication could be used by the coach in determining whether it would be better to change the player with another, for instance.

Another example of alternate applications is types of muscular training. Typically endurance training is performed at around 30% of the maximal muscular load, power training is performed at around 70% of the muscular load, whereas strength training is performed at around 90-95% of the maximal muscular load. A system such as presented above could be used to provide an indication of whether a target muscular load % is respected during training, for instance. Moreover, in the case of power training, the measurement of position can be of great use. The acceleration of the movement in relation to the weight will provide information pertaining to the power generated by the athlete. This power measurement can also be provided to optimise training and to follow the athlete's progressing to adjust his/her training plan in a manner to provide a greater progression curve.

Another example is to determine the time of reaction of an athlete in relation with a start signal. In swimming, for instance, the reaction time can be between 0.6 and 0.85 seconds on average. The difference in total time between can be between 0.1 second for 5 positions. Increasing the reaction time can thus be key between making the 1^{st} place and missing the podium. Similar comments can be made with other sprint sports. Using muscular analysis using a system or method presented herein can be useful in guiding the athlete's progression on reaction time.

As can be understood, the examples described above and illustrated are intended to be exemplary only. For instance, in different applications, a fiber electrode can be used as a sensor of EMG, as a sensor of movement, or as a sensor of both EMG and movement, to name some examples. It will also be understood that in alternate applications, rather than targeting shoulder muscles, other muscles can be targeted. In particular, the lower back can be interesting, and a system such as presented above could be easily adapted to these muscles by integrating fiber sensors into a dorsal support in the form of a belt or tight shirt, for instance. Many other muscles can be targeted in alternate embodiments. Similarly, while athletes and workers have been listed here, other individuals in similar high-demand, repetitive movement situations such as soldiers (filed operations) and elderly people (daily activities become high-demand due to again weaker muscles), or other persons undergoing rehabilitation such as persons with neurological or MSK disorders could all benefit from system. While the results were obtained using the shoulder muscles and joint, it is believed that similar results would be achievable with other joints, such as the knee, ankle, lower back, etc. In some embodiments, one or the other, or all of the indications of muscle fatigue can be somewhat absolute in nature, such as considered either "active" or "inactive". This can typically be the case of an indication which is triggered contingent upon a measured value exceeding (i.e. being above or below, depending on the specific case) a given threshold. In this sense, the composite indication of muscle fatigue can act somewhat like an "and" gate and only become active contingent upon the indications it is to be based on are all active (such as two or three, depending on the case). In some other embodiments, one or the other, or all, the indications of muscle fatigue can be represented on a scale which includes a spectrum of values, for instance. In such embodiments, individual ones of the primary indications of muscle fatigue can be weighed by a multiplication factor, for instance, which can be different for different ones of the primary indications, depending on how much a given one of the primary indication is considered reliable in terms of indication of muscle fatigue, for instance. In such cases, the composite indication of muscle fatigue can also be represented on a scale which includes a spectrum of values, such as a sum of the values of the individual indications for instance, or can be absolute in nature, such as being considered "active" if the sum of the values of the individual indications exceeds a given threshold value, for instance. Still other embodiments are possible. The scope is indicated by the appended claims.

## Claims

1. A computer-implemented method of generating an indication of muscle fatigue of a mammal wearing a fiber of an electrically conductive polymer material over a muscle having muscle fibers, the fiber extending along a fiber path having a length and a width, the length of the fiber path extending along a length of the fibers of the muscle, the method comprising :
the muscle deforming the fiber path including activating a joint of the mammal;
circulating electricity along the fiber, along the length of the fiber path;
generating a movement signal including monitoring a change of impedance of the fiber stemming from the deformation of the muscle path during said deformation of the fiber path;
determining a coefficient of correlation value of successive portions of the movement signal associated to corresponding, successive, time windows, including ascertaining a degree of similitude between the corresponding portion and a corresponding movement template;
generating an indication of muscle fatigue based on said coefficient of correlation values, wherein the indication of muscle fatigue is a first indication of muscle fatigue, a portion of the fiber forming a first electrode extends transversally relative to the length of the muscle fibers, with the first electrode maintained into electrical contact with the skin, and a second electrode maintained into electrical contact with the skin,
the method further comprising acquiring a sEMG signal from the first electrode and the second electrode, during said deformation of the fiber path, measuring a sEMG power spectrum median frequency value of successive portions of a sEMG signal within corresponding ones of the time windows and generating a second indication of muscle fatigue contingent upon said sEMG power spectrum median frequency value falling below a threshold median frequency value.

2. The method of claim 1 wherein said circulating electricity along the fiber includes circulating a DC free, AC electrical current along the fiber path.

3. The method of claim 1 wherein said generating a movement signal includes measuring an impedance using a V=RI relationship.

4. The method of claim 1 wherein said generating a movement signal includes measuring an oscillation frequency of a RLC circuit including the fiber path.

5. The method of claim 1 wherein said generating an indication of muscle fatigue is contingent upon on said coefficient of correlation value exceeding a threshold coefficient of correlation value.

6. The method of claim 1 further comprising determining a variability of said coefficient of correlation over the successive portions of the movement signal, said generating an indication of muscle fatigue is contingent upon said variability exceeding a threshold variability value.

7. The method of claim 1 wherein the movement is a repeated movement sequence, further comprising recognizing individual ones of the movements in the sequence, and defining the time windows in a manner to match a time window to each one of the movements.

8. The method of claim 7 further comprising defining the movement template based on one or more of said repeated movements.

9. The method of claim 1 wherein the time windows have a same, predetermined period of time and are immediately one after the other.

10. The method of claim 1 further comprising generating a composite indication of muscle fatigue contingent upon both the first indication of muscle fatigue and the second indication of muscle fatigue.

11. The method of claim 1 further comprising defining the threshold median frequency value based on one or more of said acquired sEMG power spectrum median frequency values.

## Patentansprüche

1. Computerimplementiertes Verfahren zum Erzeugen eines Indikators einer Muskelermüdung eines Säugetiers, das eine Faser aus einem elektrisch leitenden Polymermaterial über einem Muskel, der Muskelfasern aufweist, trägt, wobei sich die Faser entlang eines Faserpfads erstreckt, der eine Länge und eine Breite aufweist, wobei sich die Länge des Faserpfads entlang einer Länge der Fasern des Muskels erstreckt, wobei das Verfahren Folgendes umfasst:
den Muskel, der den Faserpfad verformt, einschließlich des Aktivierens eines Gelenks des Säugetiers enthält;
Zirkulieren von Elektrizität entlang der Faser entlang der Länge des Faserpfads,
Erzeugen eines Bewegungssignals einschließlich des Überwachens einer Impedanzänderung der Faser, die sich aus der Verformung des Muskelpfads während der Verformung des Faserpfads ergibt;
Bestimmen eines Korrelationskoeffizientenwerts von aufeinanderfolgenden Abschnitten des Bewegungssignals, das entsprechenden aufeinanderfolgenden Zeitfenstern zugeordnet ist, einschließlich des Überprüfens eines Ähnlichkeitsgrads zwischen dem entsprechenden Abschnitt und einer entsprechenden Bewegungsvorlage;
Erzeugen eines Indikators einer Muskelermüdung auf Basis der Korrelationskoeffizientenwerte, wobei der Indikator einer Muskelermüdung ein erster Indikator einer Muskelermüdung ist, wobei ein Abschnitt der Faser, der eine erste Elektrode bildet, sich quer zur Länge der Muskelfasern erstreckt, wobei die erste Elektrode in elektrischem Kontakt mit der Haut gehalten wird und eine zweite Elektrode in elektrischem Kontakt mit der Haut gehalten wird,
wobei das Verfahren ferner das Erfassen eines sEMG-Signals von der ersten Elektrode und der zweiten Elektrode während der Verformung des Faserpfads, das Messen eines mittleren Frequenzwerts des sEMG-Leistungsspektrums von aufeinanderfolgenden Abschnitten eines sEMG-Signals innerhalb entsprechender Zeitfenster und das Erzeugen eines zweiten Indikators einer Muskelermüdung, abhängig davon, ob der mittlere Frequenzwert des sEMG-Leistungsspektrums unter einen mittleren Frequenzschwellenwert fällt, umfasst.

2. Verfahren nach Anspruch 1, wobei das Zirkulieren von Elektrizität entlang der Faser das Zirkulieren eines gleichstromfreien elektrischen Wechselstroms entlang des Faserpfads enthält.

3. Verfahren nach Anspruch 1, wobei das Erzeugen eines Bewegungssignals das Messen einer Impedanz unter Verwendung einer V=RI-Beziehung enthält.

4. Verfahren nach Anspruch 1, wobei das Erzeugen eines Bewegungssignals das Messen einer Schwingungsfrequenz einer RLC-Schaltung einschließlich des Faserpfads enthält.

5. Verfahren nach Anspruch 1, wobei das Erzeugen eines Indikators einer Muskelermüdung davon abhängt, dass der Korrelationskoeffizientwert einen Schwellenwert für den Korrelationskoeffizientenwert überschreitet.

6. Verfahren nach Anspruch 1, das ferner das Bestimmen einer Variabilität des Korrelationskoeffizienten über die aufeinanderfolgenden Abschnitte des Bewegungssignals umfasst, wobei das Erzeugen eines Indikators einer Muskelermüdung davon abhängt, dass die Variabilität einen Schwellenwert für den Variabilitätswert überschreitet.

7. Verfahren nach Anspruch 1, wobei die Bewegung eine wiederholte Bewegungssequenz ist, ferner umfassend das Erkennen einzelner Bewegungen in der Sequenz und das Definieren der Zeitfenster, derart, dass jede der Bewegungen mit einem Zeitfenster übereinstimmt.

8. Verfahren nach Anspruch 7, das ferner das Definieren der Bewegungsvorlage auf Basis einer oder mehrerer der wiederholten Bewegungen umfasst.

9. Verfahren nach Anspruch 1, wobei die Zeitfenster eine gleiche, vorbestimmte Zeitspanne aufweisen und unmittelbar hintereinander liegen.

10. Verfahren nach Anspruch 1, das ferner das Erzeugen eines zusammengesetzten Indikators einer Muskelermüdung umfasst, abhängig sowohl von dem ersten Indikator einer Muskelermüdung als auch von dem zweiten Indikator einer Muskelermüdung.

11. Verfahren nach Anspruch 1, das ferner das Definieren des Schwellenwerts für den mittleren Frequenzwert auf Basis eines oder mehrerer der erfassten mittleren Frequenzwerte des sEMG-Leistungsspektrums umfasst.

## Revendications

1. Procédé mis en oeuvre par ordinateur de génération d'une indication de fatigue musculaire chez un mammifère portant une fibre d'un matériau polymère électroconducteur sur un muscle présentant des fibres musculaires, la fibre s'étendant le long d'un trajet de fibre présentant une longueur et une largeur, la longueur du trajet de fibre s'étendant sur une longueur des fibres du muscle, le procédé comprenant :
le muscle déformant le trajet de fibre incluant l'activation d'une articulation du mammifère ;
la circulation d'électricité le long de la fibre, le long de la longueur du trajet de fibre ;
la génération d'un signal de mouvement incluant la surveillance d'un changement d'impédance de la fibre découlant de la déformation du trajet musculaire pendant ladite déformation du trajet de fibre ;
la détermination d'un coefficient de valeur de corrélation de parties successives du signal de mouvement associé à des fenêtres temporelles successives correspondantes, incluant l'établissement d'un degré de similitude entre la partie correspondante et un modèle de mouvement correspondant ;
la génération d'une indication de fatigue musculaire sur la base dudit coefficient de valeurs de corrélation, dans lequel l'indication de fatigue musculaire est une première indication de fatigue musculaire, une partie de la fibre formant une première électrode s'étend transversalement par rapport à la longueur des fibres musculaires, la première électrode étant maintenue en contact électrique avec la peau, et une seconde électrode étant maintenue en contact électrique avec la peau,
le procédé comprenant en outre l'acquisition d'un signal sEMG provenant de la première électrode et de la seconde électrode, pendant ladite déformation du trajet de fibre, la mesure d'une valeur de fréquence médiane de spectre de puissance sEMG de parties successives d'un signal sEMG au sein de fenêtres temporelles correspondantes parmi les fenêtres temporelles et la génération d'une seconde indication de fatigue musculaire dépend de ladite valeur de fréquence médiane de spectre de puissance sEMG tombant au-dessous d'une valeur de fréquence médiane seuil.

2. Procédé selon la revendication 1 dans lequel ladite circulation d'électricité le long de la fibre inclut la circulation d'un courant électrique CA exempt de CC le long du trajet de fibre.

3. Procédé selon la revendication 1 dans lequel ladite génération d'un signal de mouvement inclut la mesure d'une impédance en utilisant une relation V=RI.

4. Procédé selon la revendication 1 dans lequel ladite génération d'un signal de mouvement inclut la mesure d'une fréquence d'oscillation d'un circuit RLC incluant le trajet de fibre.

5. Procédé selon la revendication 1 dans lequel ladite génération d'une indication de fatigue musculaire dépend dudit coefficient de valeur de corrélation dépassant un coefficient seuil de valeur de corrélation.

6. Procédé selon la revendication 1 comprenant en outre la détermination d'une variabilité dudit coefficient de corrélation sur les parties successives du signal de mouvement, ladite génération d'une indication de fatigue musculaire dépendant de ladite variabilité dépassant une valeur de variabilité seuil.

7. Procédé selon la revendication 1 dans lequel le mouvement est une séquence de mouvements répétés, comprenant en outre la reconnaissance de mouvements individuels des mouvements dans la séquence, et la définition des fenêtres temporelles afin de faire correspondre une fenêtre temporelle avec chacun des mouvements.

8. Procédé selon la revendication 7 comprenant en outre la définition du modèle de mouvement sur la base d'un ou de plusieurs desdits mouvements répétés.

9. Procédé selon la revendication 1 dans lequel les fenêtres temporelles présentent une même durée prédéterminée et sont les unes à la suite des autres.

10. Procédé selon la revendication 1 comprenant en outre la génération d'une indication composite de fatigue musculaire dépendant à la fois de la première indication de fatigue musculaire et de la seconde indication de fatigue musculaire.

11. Procédé selon la revendication 1 comprenant en outre la définition de la valeur de fréquence médiane seuil sur la base d'une ou de plusieurs desdites valeurs de fréquence médiane de spectre de puissance sEMG acquises.
